(19) **Europäisches Patentamt / European Patent Office / Office européen des brevets**

(11) **EP 2 280 718 B1**

(12) **EUROPEAN PATENT SPECIFICATION**

(45) Date of publication and mention
of the grant of the patent:
**27.07.2016 Bulletin 2016/30**

(51) Int Cl.:
*A61Q 19/08* (2006.01)     *A61Q 19/00* (2006.01)
*A61K 8/42* (2006.01)      *A61K 8/97* (2006.01)
*A61K 36/15* (2006.01)     *A61K 8/88* (2006.01)

(21) Application number: **09726458.4**

(22) Date of filing: **01.04.2009**

(86) International application number:
**PCT/CA2009/000379**

(87) International publication number:
**WO 2009/121168 (08.10.2009 Gazette 2009/41)**

(54) **EXTRACTS FROM PLANTS OF THE TSUGA GENUS AND USES THEREOF IN THE TREATMENT OF INFLAMMATION, IRRITATION AND/OR INFECTION**

EXTRAKTE VON PFLANZEN DES GENUS TSUGA UND IHRE ANWENDUNG IN DER BEHANDLUNG VON ENTZÜNDUNGEN, REIZUNGEN UND/ODER INFEKTIONEN

EXTRAITS DE PLANTES DU GENRE TSUGA ET UTILISATIONS DE CEUX-CI DANS LE TRAITEMENT DE L'INFLAMMATION, DE L'IRRITATION ET/OU DE L'INFECTION

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **01.04.2008 US 41553 P**

(43) Date of publication of application:
**09.02.2011 Bulletin 2011/06**

(73) Proprietor: **Lucas Meyer Cosmetics Canada Inc.**
**Québec, QC G1V 4W2 (CA)**

(72) Inventors:
• **GUAY, Johane**
  **Quebec G3A 2L7 (CA)**
• **CYR, Benoit**
  **Québec G3A 2H2 (CA)**
• **GENDRON, Nathalie**
  **Neuville**
  **Quebec G0A 2R0 (CA)**
• **PAGE, Brigitte**
  **Quebec**
  **Quebec G2B 3P7 (CA)**

(74) Representative: **Leonard, Thomas Charles et al**
**Kilburn & Strode LLP**
**20 Red Lion Street**
**London WC1R 4PJ (GB)**

(56) References cited:
**WO-A1-2006/039807     WO-A1-2006/053415**
**US-A- 6 130 254**

• **DATABASE WPI Week 200825 Thomson Scientific, London, GB; AN 2008-D28645 XP002698972, & CN 101 049 301 A (HUBEI COLLEGE TRADITIONAL CHINESE MEDICI) 10 October 2007 (2007-10-10)**
• **TURNER N J ET AL: "Contemporary use of bark for medicine by two salishan native elders of Southeast Vancouver Island, Canada", JOURNAL OF ETHNOPHARMACOLOGY, ELSEVIER SCIENTIFIC PUBLISHERS LTD, IE, vol. 29, no. 1, 1 April 1990 (1990-04-01), pages 59-72, XP25502351, ISSN: 0378-8741, DOI: 10.1016/0378-8741(90)90098-E [retrieved on 1990-04-01]**
• **MCCUNE L M ET AL: "Symptom-specific antioxidant activity of boreal diabetes treatments", PHARMACEUTICAL BIOLOGY, SWETS AND ZEITLINGER, LISSE, NL, vol. 41, no. 5, 1 January 2003 (2003-01-01), pages 362-370, XP009150909, ISSN: 1388-0209, DOI: 10.1076/PHBI.41.5.362.15942**
• **DATABASE WPI Week 199005 Thomson Scientific, London, GB; AN 1990-033364 XP002698992, & JP H01 311020 A (SHISEIDO CO LTD) 15 December 1989 (1989-12-15)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PURWAR R. ET AL.: 'Modulation of keratinocyte-derived MMP-9 by IL-13: a possible role for the pathogenesis of epidermal inflammation' JOURNAL OF INVESTIGATIVE DERMATOLOGY vol. 128, 2008, pages 59 - 66, XP008146397**
- **ELKINGTON P.T.G. ET AL.: 'The paradox of matrix metalloproteinases in infectious disease' CLINICAL AND EXPERIMENTAL IMMUNOLOGY vol. 142, 2005, pages 12 - 20, XP008146400**

Remarks:
    The file contains technical information submitted after the application was filed and not included in this specification

**Description**

**FIELD OF THE INVENTION**

The present invention relates to fields of pharmaceuticals and cosmetics and, in particular, to extracts from the *Tsuga canadensis* plant for use to treat microbial infection of the skin associated with acne.

**BACKGROUND OF THE INVENTION**

The skin performs multiple functions such as protection, barrier, temperature control, excretion and also respiration. It is the main target tissue since it is exposed to all environmental hazards. Not only is the skin subjected to germs, toxic chemicals and hostile environments, it is the only organ directly exposed to ultraviolet light (UV). Over time, physiological changes occur to this organ and lead to a decrease in the functionality of the skin. Changes that occur with ageing, for example, include decrease in thickness, loss of moisture, sagging, loss of elasticity, age spots and wrinkles. Hence, a variety of dermatological conditions may occur as a result of ongoing intrinsic factors (for example, chronological ageing, disease and allergies) and/or exposure to a number of extrinsic factors (such as infection, trauma, radiation, toxins and steroid use).

A number of these dermatological conditions are the result of an inflammatory response or include an inflammation component. Skin irritation is probably one of the most common adverse effects resulting from inflammatory reactions in humans. For example, UV light, allergens, exogenous stress and products found in dermatological formulations such as surfactants are all known to induce inflammatory reactions in the epidermis (Welss T, Toxicology in vitro 18:231, 2004).

The use of retinol and its derivatives in skin care products, for example, has many beneficial effects, however, the concentrations of these compounds that may be used is limited because of the severe local irritation, manifested as mild erythema and stratum corneum peeling of the skin, that the compounds induce (Kim BH, Toxicology Letters 146:65, 2003). Retinol and its derivatives are widely used to treat or ameliorate acne, psoriasis, keratinisation disorders and cutaneous malignancies (Boehm B, Exp Opinion Invest Drugs 4:593, 1995). They are also used in cosmetic formulations to reduce wrinkles (Varani J, J Invest Dermatol Symp 3:57, 1998) and improve the appearance of cellulite (Kligman AM, J Dermatol Threat 10:119, 1999). AHAs (alpha hydroxy acids) are widely used in facial peeling preparations and in anti-ageing and anti-acne dermatological formulations, are also known to induce irritation after treatment and/or prolonged exposure. Other products such as kojic acid, which are used as whitening agents in skin preparations, have likewise been reported to have highly sensitising effects and may cause irritation at their active concentrations.

Psoriasis is a common chronic, recurrent auto-immune disease of the skin characterized by dry, well-circumscribed, silvery, scaling papules and plaques of various sizes. Psoriasis varies in severity from one or two lesions to widespread dermatosis, sometimes associated with disabling arthritis or exfoliation. Psoriasis is a complex disease; its cause is unknown, but the thick scaling has traditionally been attributed to increased epidermal cell proliferation and concomitant dermal inflammation. Macroscopically, psoriasis is characterized by underlying skin redness (inflammation and accompanying angiogenesis), with overlying keratinocyte hyperproliferation.

Extracts from plants and specific compounds obtained from plant sources are often used in cosmetic and pharmaceutical compositions. For many years in various cultures, medicinal plant extracts have been used for treatment of certain disorders and as cosmetics. For example, Aloe vera promotes a variety of anti-inflammatory responses in the body, reducing swelling from injuries and promoting recovery from infections. Such anti-inflammatory responses not only aid in the relief of pain and discomfort, but also enhance the overall wound healing process. Chamomile is known to improve tissue regeneration, reduce inflammation and encourage the healing of wounds. Flavonoids such as apigenin as well as a distinctive blue essential oil (azulene) derived from chamomile have been found to reduce inflammation and encourage the healing of wounds. *Salix alba* (willow bark) extract is a natural source of salicylic acid, which is a well-known anti-inflammatory product.

Topical skin applications are known in the art to help shield the skin from the vagaries of the environment. Conventional skin protection typically attempts to either protect the skin from UV light (see U.S. Patent No. 5,141,741) or provide additional agents capable of neutralizing free radicals (U.S. Patent No. 6,764,693). Methods of inhibiting either chronological or photo-ageing of the skin by application of UV blocking compounds in combination with compounds that inhibit MMPs have also been reported (U.S. Patent Nos. 5,837,224; 6,130,254 and 6,365,630 and U.S. Patent Application Publication No. 20010053347). Mercaptoketone and mercaptoalcohol compounds that inhibit the activity of MMPs and their use in treating or controlling disease states such as arthropathy, dermatological conditions, bone resorption, inflammatory diseases and tumor invasion have also been described (U.S. Patent No. 6,307,101).

Addition of certain plant extracts or phyto-compounds to preparations, such as lotions, creams and gels, to treat dermatological disorders has also been reported. These cosmetic compositions serve to shield the skin from UV light (U.S. Patent Nos. 4,857,325; 5,141,741 and 6,342,208) and act as antioxidants in the neutralization of free radicals (U.S. Patent No. 4,923,697). Some fruit extract-containing dermatological agents, capable of neutralizing free radicals, additionally moisturize and facilitate the hydration of the skin (see U.S. Patent No. 6,800,292).

Other plant extracts useful in dermo-cosmetics have been described (see U.S. Patent Nos. 6,682,763; 5,824,320 and 6,406,720). Here, external agents derived from olive plants are reported as having skin-beautifying effects, in particular, an anti-ageing effect related to the prevention and elimination of wrinkles and sags of the skin (U.S. Patent No. 6,682,763). Furthermore, a whitening effect, which can lighten (U.S. Patent No. 5,073,545) or prevent dark skin, melasma, ephelis and darkening or dullness of the skin has been reported (U.S. Patent No. 6,682,763). Plant extracts useful in the treatment of eczema and/or psoriasis (U.S. Patent Nos. 6,676,975 and 4,855,131), and for maintaining general skin care (U.S. Patent No. 6,193,975) have also been described.

International Patent Application No. PCT/CA04/02007 (WO 2006/053415) describes a large number of plant extracts that are useful for the preparation of dermatological formulations and uses of these formulations for ameliorating the effects of ageing and for the routine care of skin, hair and/or nails.

The *Tsuga* genus is a genus of conifers in the family Pinaceae. Plants in this genus are known under the common name of "hemlock." Catechol tannins extracted from *Tsuga* or hemlock have been described for the treatment of burns (U.S. Patent No. 2,276,241; GB Patent No. 544,615 and Canadian Patent No. 406,408) due to their tanning action. As further described in these patents, tannins are not germicidal and as such the burn treatment compositions further comprise an effective germicide, specifically a phenolic compound, which is compatible with the tannin.

*Tsuga* extracts have been described for their deodorant properties. For example, Japanese Patent Application Publication No. 2002087973 describes extracts from *Tsuga* as part of cosmetic compositions for suppressing human body odour; Japanese Patent Application Publication No. 4030855 describes a mousse-like deodorant containing several plant extracts including a *Tsuga* extract, and U.S. Patent No. 4,898,727 describes a deodorant containing several plant extracts including a *Tsuga* extract, a filter using same and a method of producing the deodorant.

European Patent Application Publication No. 0 870 507 describes a synergistic anti-bacterial composition that includes an extract of botanical materials and an essential oil. The essential oil is described as having anti-microbial activity, whereas the extract of botanical materials has significantly lower activity, or no anti-microbial activity, when used alone. A variety of potential botanical materials are described in the application including *Tsuga,* with the preferred material being a combination of Plantago, Hypericum, Echinacea and Propolis.

This background information is provided for the purpose of making known information believed by the applicant to be of possible relevance to the present invention. No admission is necessarily intended, nor should be construed, that any of the preceding information constitutes prior art against the present invention.

## SUMMARY OF THE INVENTION

The subject matter for which protection is sought is as defined in the claims. In particular, the present invention provides a butylene glycol:water 50:50 (v/v) extract from needles, twigs or small branches of a *Tsuga canadensis* plant, for use to treat microbial infection of the skin in a subject in need thereof, wherein the microbial infection of the skin is associated with acne.

The present invention also provides a dermatological formulation comprising an extract as defined in any one of claims 1 to 5, in combination with one or more other therapeutic or cosmetic agents, for use according to claim 1.

## BRIEF DESCRIPTION OF THE FIGURES

These and other features of the invention will become more apparent in the following detailed description in which reference is made to the appended drawings.

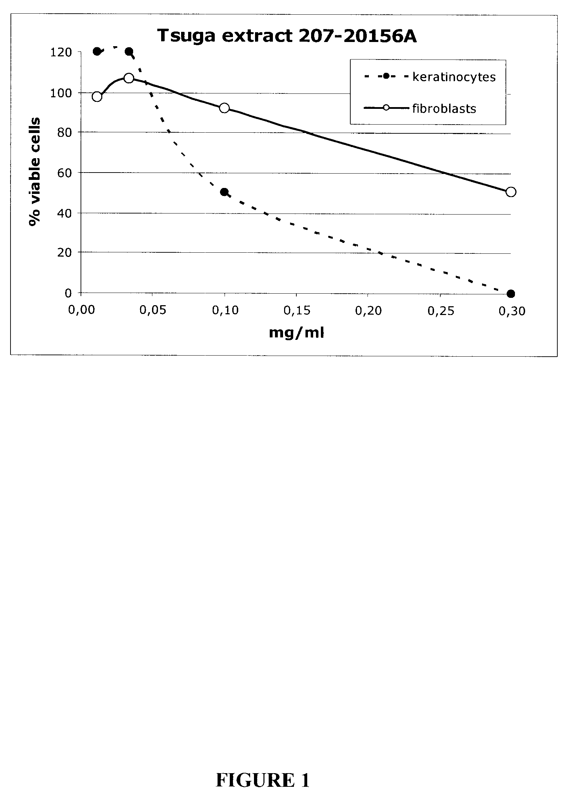

**Figure 1** presents a graph illustrating the viability of skin cells treated with different concentrations of a *Tsuga canadensis* extract in accordance with one embodiment of the invention.

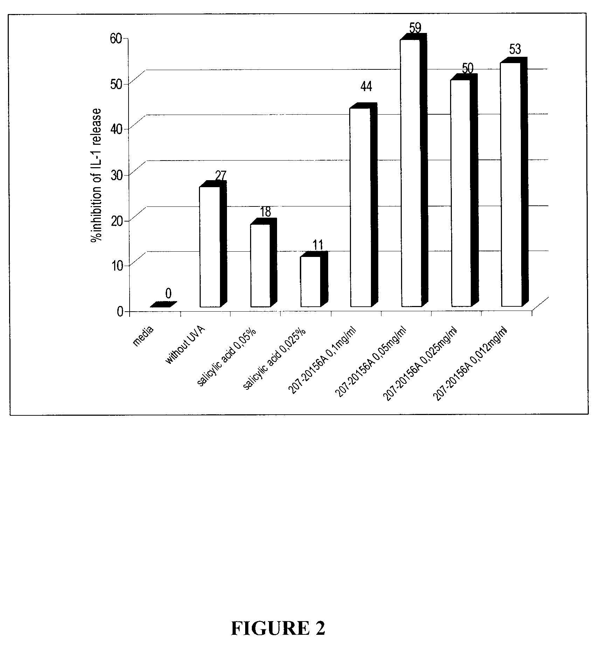

**Figure 2** illustrates the anti-inflammatory effect of a *Tsuga canadensis* extract in accordance with one embodiment of the invention. The bar graph depicts the inhibition of UVA-induced interleukin-1 (IL-1) release in human keratinocytes *in vitro* by different concentrations of the *Tsuga canadensis* extract "207-20156A".

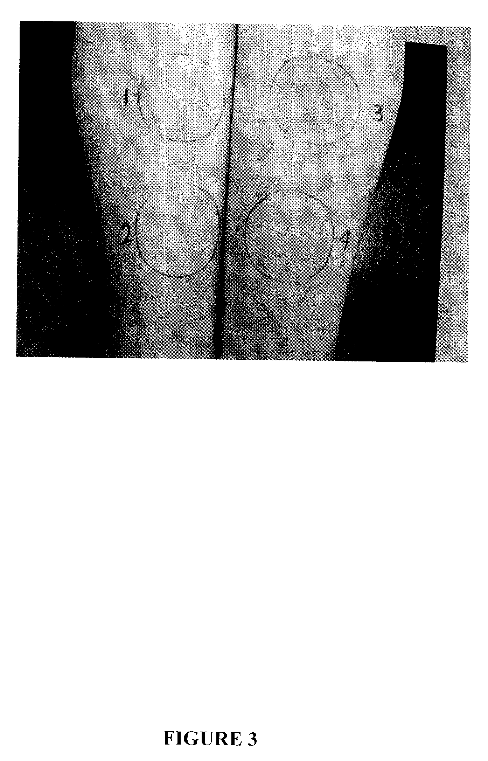

**Figure 3** presents a photograph of areas of the skin of a human volunteer that have been treated with a *Tsuga* extract in accordance with one embodiment of the invention and demonstrates the anti-inflammatory effect of the *Tsuga canadensis* extract on retinol-induced inflammation of the skin *in vivo.* Area 1 was treated with a cream containing 0.5% (w/w) retinol; Area 2 was treated with a cream containing 0.5% (w/w) retinol and 5% (w/w) of the *Tsuga* extract; Area 3 was treated with a cream containing 1% (w/w) retinol, and Area 4 was treated with a cream containing 0.5% (w/w) retinol and 5% (w/w) of the *Tsuga* extract.

## DETAILED DESCRIPTION OF THE INVENTION

The present disclosure relates to the newly identified anti-inflammatory and anti-microbial properties of extracts derived from plants of the *Tsuga* genus (*"Tsuga* extracts"). In its broadest aspect, therefore, the present disclosure provides for the use of the *Tsuga* extracts to ameliorate inflammation, irritation and/or infection.

In accordance with the present disclosure, the *Tsuga* extracts are also capable of inhibiting one or more of angiogenesis, contractile force of fibroblasts and/or UV-induced protease activity. These properties, together with the anti-inflammatory and anti-microbial properties of the *Tsuga* extracts, render the extracts well-suited for the treatment of dermatological conditions with an associated inflammatory component or infection (such as, for example, psoriasis, rosacea, erythema and acne); for combating the irritant or inflammatory effects of other skin treatment compounds (such as retinol); for combating the irritant or inflammatory effects of environmental factors (such allergens or over-exposure to the sun); for treating obesity-related skin problems (for example, inflammation, redness, erythema, rashes and/or bacterial infections caused by skin folds); for combating the irritant or inflammatory effects of, or infection due to, cosmetic or surgical skin procedures, such as peels, exfoliation, laser treatments, hair removal, plastic surgery and the like; for treating irritation, inflammation and/or infection associated with diaper rash; for incorporation into dermatological formulations for sensitive skins, and for providing a preservative effect to dermatological formulations, as well as for ameliorating the dermatological effects of ageing.

In one aspect, the present invention provides for the use of *Tsuga canadensis* plant extracts for treatment of microbial skin infection, associated with acne. In another aspect, the invention provides for the use of such extracts in dermatological formulations for the treatment of microbial skin infection associated with acne in order to combat the irritant or inflammatory effects of other components of the formulation on the skin, for example, to render the formulation more amenable for sensitive skins or to combat the irritant effect of retinol or other irritants or sensitizing agents in skin care preparations, such as anti-ageing or anti-acne formulations. In this context, the *Tsuga* extract may be included in the skin care preparation or may be applied separately from the skin care preparation. In a further aspect, the disclosure provides for the use of the *Tsuga* extract in dermatological formulations for ameliorating the irritant/inflammatory effects of environmental factors, for example in "after sun" formulations. In another aspect, the present disclosure provides for the use of the *Tsuga* extract in dermatological formulations for providing a soothing or healing effect to inflamed, irritated or infected skin, for example, resulting from obesity-related skin problems, from exfoliation, hair removal, laser treatments or diaper rash. In another aspect, the invention provides for the use of the *Tsuga* extract in dermatological formulations in order to provide a preservative effect to the formulation. In a further aspect, the invention provides for the use of the *Tsuga* extracts as anti-ageing or anti-acne agents.

The ability of the *Tsuga* extracts to combat the irritant effects of retinol, retinol derivatives and other irritants, such as alpha-hydroxy acids (AHAs) and skin whitening agents, allows for the use of formulations that comprise the extract and higher than standard amounts of these agents. In one embodiment, therefore, the present invention provides for dermatological formulations that comprise a *Tsuga* extract and one or more of retinol, a retinol derivative, an AHA or a skin whitening agent, wherein the retinol, retinol derivative or AHA is present in a higher than standard amount. In another embodiment, the present invention provides for dermatological formulations that comprise a *Tsuga* extract and standard amounts of one or more of retinol, a retinol derivative, an AHA or a skin whitening agent. The dermatological compositions comprising a *Tsuga* extract and retinol, a retinol derivative, or an AHA may be used, for example, to ameliorate the dermal signs of ageing or to treat acne.

*Definitions*

Unless defined otherwise, all technical and scientific terms used herein have the same meaning as commonly understood by one of ordinary skill in the art to which this invention belongs.

The term "plant material," as used herein, refers to any part or parts of a specified plant taken either individually or in a group. Examples include, but are not limited to, leaves, needles, roots, bark, stems, buds, twigs, cones, branches and the like.

The term "extract," as used herein with reference to a specified plant, refers to a composition prepared by contacting plant material with a solvent following the procedures described herein. The extract can optionally be subjected to one or more separation and/or purification steps.

The term "isolated," as used herein in the context of an isolated compound (or active ingredient), refers to a compound that is in an environment different from that in which the compound naturally occurs. "Isolated" is meant to include compounds that are within samples that are substantially enriched for the compound of interest and/or in which the compound of interest is partially or substantially purified.

The term "substantially pure," as used herein refers to a compound (or active ingredient) that is removed from its natural environment and that constitute at least about 50% of a sample, for example at least about 60%, at least about 70%, at least about 80% or at least about 90% of a sample.

The term "skin cell," as used herein, refers to a cell normally present within the skin of a mammal and includes, but is not limited to, keratinocytes, fibroblasts, endothelial cells (including vascular endothelial cells), basal cells, granular cells, Merkel cells, melanocytes, Langerhans cells, leukocytes, mastocytes, nerve cells, adipose cells and macrophages.

The term "attenuate," as used herein, means to reduce or inhibit, wherein the inhibition may be complete or partial inhibition.

The term "cell migration," as used herein, refers to the movement, typically abnormal, of a cell or cells from one locus to another. Examples of cell migration include the movement of endothelial cells during angiogenesis.

A "dermatological formulation," as used herein, refers to a pharmaceutical composition or a cosmeceutical composition formulated for topical administration to the skin. In one embodiment, the dermatological formulation is for administration to a portion or portion of the skin affected by a dermatological condition or disorder.

The term "dermatological condition," as used herein, refers to a condition, such as a disease, disorder, irritation, reaction and the like, present in the skin of a subject that is caused by intrinsic or extrinsic factors and/or by ageing.

The term "ameliorate," as used herein, means to make more tolerable (for example by reducing the incidence or severity), to heal or to cure.

The term "treatment," as used herein, refers to an intervention performed with the intention of improving a recipient's status. The improvement can be subjective or objective and is related to the amelioration, either temporary or long-term, of one or more of the symptoms associated with a condition being treated. In some embodiments, treatment includes the prevention of the development of the condition. Thus, in various embodiments, the term treatment includes the prevention (prophylaxis), moderation, reduction, and/or curing of a condition at various stages. In certain embodiments, prevention of deterioration of a recipient's status is also encompassed by the term. Those in need of treatment thus may include those already having the condition as well as those prone to, or at risk of developing, the condition and those in whom the condition is to be prevented.

The term "subject," as used herein, refers to an individual in need of treatment or who would otherwise benefit from the use of a dermatological formulation in accordance with the invention.

As used herein, the term "about" refers to approximately a +/-10% variation from a given value. It is to be understood that such a variation is always included in any given value provided herein, whether or not it is specifically referred to.

## *TSUGA* EXTRACTS

The present disclosure provides for extracts from plants of the *Tsuga* genus *("Tsuga* extracts")* suitable for dermatological use. In accordance with the present disclosure, the *Tsuga* extracts have anti-inflammatory and anti-microbial activity. In the present disclosure, the *Tsuga* extracts can also be capable of inhibiting one or more of angiogenesis, contractile force of fibroblasts or activity of UV-induced proteases.

In accordance with the present disclosure, the *Tsuga* extracts are solvent-based extracts obtained by solvent extraction of plant material from a selected *Tsuga* plant. The selected *Tsuga* plant can be, for example, *Tsuga canadensis; Tsuga caroliniana; Tsuga chinensis; Tsuga diversifolia; Tsuga dumosa; Tsuga forrestii; Tsuga heterophylla; Tsuga mertensiana* or *Tsuga sieboldii.* According to the present invention, the *Tsuga* plant is a plant native to North America, *i.e. Tsuga* canadensis.

The solvent used for the preparation of the extract can be an aqueous solvent (such as water or a buffer), or it can be a liquid organic compound, or a combination of an aqueous solvent and a liquid organic compound. In some embodiments, the solvent may be a supercritical or sub-critical fluid. In one embodiment the *Tsuga* extract is an aqueous, alcoholic or aqueous-alcoholic extract. In another embodiment, the *Tsuga* extract is an aqueous, glycolic or aqueous-glycolic extract.

In embodiments of the invention, the extract is a butylene glycol:water 50:50 (v/v) extract from needles, twigs or small branches of a *Tsuga canadensis* plant.

## PREPARATION OF THE *TSUGA* EXTRACTS

The *Tsuga* extracts in accordance with the invention are obtained by solvent extraction of plant material from a *Tsuga canadensis* plant.

### *Plant Material*

The plant material is derived from *Tsuga canadensis.* The *Tsuga* extract is prepared from needles, twigs, small branches or any combination thereof.

The plant material can be fresh, dried or frozen. In one embodiment, the plant material used in the preparation of the *Tsuga canadensis* extracts is dried. The plant material may be used immediately after harvesting or it can be stored for a period of time prior to being subjected to the extraction process. If the plant material is stored, it can be treated prior to storage, for example, by drying, freezing, lyophilizing, or some combination thereof, as is known in the art. The storage time may be of various durations, for example, the storage period may be between a few days and a few years. Typically storage times range between less than one week and about one year in duration.

If desired, the plant material can be treated prior to the extraction process in order to facilitate the extraction process. Typically such treatment results in the plant material being fragmented by some means such that a greater surface area is presented to the solvent. For example, the plant material can be crushed or sliced mechanically, using a grinder or other device to fragment the plant parts into small pieces or particles, or the plant material can be frozen in liquid nitrogen and then crushed or otherwise fragmented into smaller pieces.

If desired and when practicable, the plant material can be derived from a *Tsuga canadensis* plant that was subjected to a stress treatment. A stress treatment comprises contacting or treating the plant, or material from the plant, with one or more stressor with the aim of inducing or eliciting increased production of one or more chemicals. The stressor can be a chemical compound or a physical treatment. Examples of chemical stressors include, but are not limited to, organic and inorganic acids including fatty acids; glycerides; phospholipids; glycolipids; organic solvents; amino acids; peptides; monosaccharides; oligosaccharides; polysaccharides; lipopolysaccharides; phenolics; alkaloids; terpenes; terpenoids; antibiotics; detergents; polyamines; peroxides; ionophores, and the like. Examples of physical stress treatments include, but are not limited to, ultraviolet radiation, sandblasting, low and high temperature stress, and osmotic stress induced by salt or sugars. Nutritional stress is another example of a physical stress and is defined as depriving the plant of essential nutrients (*e.g.* nitrogen, phosphorus or potassium) in order to induce or elicit increased production of one or more chemicals. The one or more stressor (*i.e.* chemical compound(s), physical treatment(s), or combination thereof) may be applied continuously or intermittently to the plant material. Various stressors and procedures for stressing plants prior to extract preparation have been described previously (see International Patent Application WO 02/06992) and are suitable for use in accordance with the present invention.

### *Solvent extraction*

Various extraction processes are known in the art and can be employed in the process of the present disclosure (see, for example, International Patent Application WO 02/06992). The solvent extraction process employed in the preparation of the *Tsuga* extracts typically employs as solvent an aqueous solvent (such as water or a buffer), a liquid organic compound, or a combination thereof. Exemplary liquid organic compounds that can be used as solvents in the extraction process to prepare the *Tsuga* extracts include, but are not limited to, alcoholic solvents, which include primary alcohols such as methyl alcohol (methanol), ethyl alcohol (ethanol), 1-propanol and 1-butanol; secondary alcohols such as 2-propanol and 2-butanol; tertiary alcohols such as 2-methyl-2-propanol, and liquid polyhydric alcohols such as glycerine (glycerol) and glycols. Suitable glycols include, for example, ethylene glycol (1,2-ethandiol), propylene glycol (1,2-propanediol), trimethylene glycol (1,3-propanediol), 1,3-butylene glycol, pentylene glycol (1,2-pentanediol), hexylene glycol (2-methyl-2,4-pentanediol), diethylene glycol, dipropylene glycol and lower molecular weight polyethylene glycols. Other known organic solvents for plant extraction include acetone, tetrahydrofuran, acetonitrile, 1,4-dioxane, pyridine, dimethylsulfoxide, N,N-dimethyl formamide, acetic acid, diethyl ether, hexane, heptane, dichloromethane and ethyl acetate. Supercritical or sub-critical fluids, such as water or carbon dioxide, are also suitable solvents for the preparation of the *Tsuga* extracts.

In the embodiment of the invention, the solvent employed to prepare the *Tsuga* canadensis extracts comprises an alcohol, namely butylene glycol. In an embodiment, of the disclosure the solvent employed to prepare the *Tsuga* extracts comprises a primary alcohol or a liquid polyhydric alcohol. In another embodiments, of the disclosure the solvent employed to prepare the *Tsuga* extracts comprises a supercritical or sub-critical fluid.

When the extraction process is carried out using a solvent that comprises a mixture of an aqueous solvent and a liquid organic compound, the content of the liquid organic compound ranges from about 5% to about 95% by volume. In one embodiment of the disclosure, the extraction process is carried out using a solvent that comprises a mixture of an aqueous solvent and a liquid organic compound in which the content of the liquid organic compound ranges from about 10% to about 95% by volume. In another embodiment, the extraction process is carried out using a solvent that comprises a mixture of an aqueous solvent and a liquid organic compound in which the content of the liquid organic compound ranges from about 15% to about 95% by volume. In other embodiments, the extraction process is carried out using a solvent that comprises a mixture of an aqueous solvent and a liquid organic compound in which the content of the liquid organic compound ranges from about 10% to about 90% by volume, from 20% to about 95% by volume, from about 20% to about 90% by volume, from about 10% to about 85% by volume and from about 20% to about 85% by volume.

In one embodiment of the present disclosure, a solvent that is compatible with mammalian skin is used in the extraction. This can, for example, allow for the extract to be incorporated directly into a dermatological formulation with little, or no, further processing. Examples of such solvents include, but are not limited to, water, an aqueous buffer, a combination of water/buffer with a lower alcohol or an anhydrous lower alcohol. In the context of the present invention, a lower alcohol refers to an alcohol having 1 to 6 carbon atoms, such as a primary, secondary, tertiary or liquid polyhydric alcohol. In one embodiment of the present disclosure, the solvent for the preparation of the *Tsuga* extract is selected from water, a lower alcohol or a combination thereof. In another embodiment, the solvent for the preparation of the *Tsuga* extract comprises a lower alcohol selected from the group of: methyl alcohol (methanol), ethyl alcohol (ethanol), 1-propanol, 1-butanol, 2-propanol, 2-butanol, 2-methyl-1-propanol, 2-methyl-2-propanol, glycerine, ethylene glycol, propylene glycol, diethylene glycol, dipropylene glycol, 1,3-propanediol and 1,3-butylene glycol.

When the extraction process employs a combination of an aqueous solvent and a lower alcohol as solvent, the lower alcohol content of the solvent typically ranges from about 5% to about 95% by volume, for example from about 10% to about 95% by volume. In one embodiment of the disclosure, the extraction process is carried out using a solvent that comprises a mixture of an aqueous solvent and a lower alcohol in which the content of the lower alcohol ranges from about 15% to about 95% by volume. In another embodiment of the disclosure, the extraction process is carried out using a solvent that comprises a mixture of an aqueous solvent and a lower alcohol in which the content of the lower alcohol ranges from about 20% to about 95% by volume. In other embodiments, the extraction process is carried out using a solvent that comprises a mixture of an aqueous solvent and a lower alcohol in which the content of the lower alcohol ranges from about 20% to about 90% by volume, and from about 20% to about 85% by volume.

For example, when the extraction process employs a solvent that is an aqueous solvent/primary alcohol mixture, the primary alcohol can be present in an amount between about 20% to about 90% by volume, for example from about 30% to about 90% by volume, whereas when the extraction process employs a solvent that is an aqueous solvent/glycol mixture, the glycol can be present in an amount between about 10% to about 80% by volume, for example from about 10% to about 60% by volume. Similarly, when the extraction process employs a solvent that is an aqueous solvent/glycerine mixture, the glycerine can be present in an amount between about 10% to about 80% by volume, for example from about 10% to about 60% by volume.

In one embodiment of the disclosure, the extraction process employs a solvent that is an aqueous solvent/primary alcohol mixture in which the primary alcohol is present in an amount between about 35% to about 90% by volume. In another embodiment, the extraction process employs a solvent that is an aqueous solvent/primary alcohol mixture in which the primary alcohol is present in an amount between about 40% to about 90% by volume. In another embodiment, the extraction process employs a solvent that is an aqueous solvent/primary alcohol mixture in which the primary alcohol is present in an amount between about 45% to about 90%, between about 45% to about 85%, and between about 50% to about 85% by volume.

In an alternate embodiment of the disclosure, the extraction process employs a solvent that is an aqueous solvent/glycol mixture in which the glycol is present in an amount between about 15% to about 60% by volume. In another embodiment, the extraction process employs a solvent that is an aqueous solvent/glycol mixture in which the glycol is present in an amount between about 15% to about 55% by volume. In another embodiment, the extraction process employs a solvent that is an aqueous solvent/glycol mixture in which the glycol is present in an amount between about 20% to about 55%, and between about 20% to about 50% by volume.

In embodiments of the invention, the extraction process employs butylene glycol:water 50:50 (v/v). A number of standard extraction techniques known in the art can be employed to prepare the plant extracts. In general, the extraction process entails contacting solid plant material with a solvent with adequate mixing and for a period of time sufficient to ensure adequate exposure of the solid plant material to the solvent such that activity present in the plant material can be taken up by the solvent.

An appropriate amount of the solvent to be used in the extraction can be determined by the skilled worker based on the amount of plant material being employed in the extraction. In one embodiment of the invention, the w/v (g/100mL) of plant material to solvent used in the extraction process is between about 1/2 and about 1/50. In another embodiment, the w/v (g/100mL) of plant material to solvent used in the extraction process is between about 1/5 and about 1/50. In another embodiment, the w/v (g/100mL) of plant material to solvent used in the extraction process is between about 1/10 and about 1/50. In other embodiments, the w/v (g/100mL) of plant material to solvent used in the extraction process is between about 1/10 and about 1/40; between about 1/10 and about 1/30; and between about 1/10 and about 1/25.

A variety of conditions can be employed for the extraction process. Typically, the extraction procedures are conducted over a period of time between about 10 minutes and about 72 hours at a temperature between about 4°C and about 50°C. However, temperatures between about 4°C and about 90°C, for example between about 4°C and about 70°C can be employed. Higher temperatures are also contemplated, with or without increased pressure, when certain extraction techniques are employed, for example, pressurised liquid extraction, sub-critical fluid extraction (for example, sub-critical water extraction (SWE)) or supercritical fluid extraction. Similarly, the extraction time may be varied depending on other extraction conditions, such as the solvent and temperature employed, for example, the extraction time can range from several minutes to several days. For example, in one embodiment, the extraction time is at least one hour. In another embodiment, the extraction time is between about one hour and about 72 hours. Determination of appropriate extraction temperatures and times is within the ordinary skills of a worker in the art.

Adequate contact between the solvent and the plant material can be encouraged by shaking, stirring, percolating and/or macerating the suspension. Alternatively, an extraction device equipped with, for instance, a stirring machine, or a soxhlet or other device known in the art can be employed which may improve the extraction efficiency. The extraction can be carried out at ordinary pressure, under pressure or at reduced pressure established by, for example, aspiration. Appropriate extraction conditions can readily be determined or selected by one skilled in the art taking into consideration the production conditions such as production facilities and yields.

The present disclosure also provides for the use of supercritical fluid extraction for the preparation of the *Tsuga* extracts. Supercritical fluid extraction involves the use of a supercritical fluid (SCF) as a solvent. A SCF is a liquid or a gas at atmospheric conditions, but becomes supercritical when it is compressed above its critical pressure and heated above its critical temperature. Supercritical fluids have increased dissolving power in their supercritical regions. A supercritical fluid exhibits properties between those of a gas and a liquid, and has the capacity to dissolve compounds that may only dissolve poorly or not at all in the gas or liquid state. Most components extracted from the plant material, once dissolved, can quickly and cleanly be precipitated or removed from the supercritical fluids by lowering the pressure and/or temperature to achieve separation. Using the method of post-extraction fractionation with a column designed to allow for temperature and pressure drops at different levels to gain the desired results may effect further concentration and purification when desirable.

Supercritical fluid extraction processes are well known in the art, for example, see Martinez, J.L. (supercritical Fluid Extraction of Nutraceuticals and Bioactive Compounds (2007, CRC Press, Boca Raton, FL) and Herrero. M. et al. (2005, Food Chem, 98:136-148).

In general, the starting plant material is placed in an extractor device together with the supercritical fluid, with or without a chemical modifier, at specified pressure and temperature conditions to extract the desired components from the plant material. After extraction, the fluid and the compound are passed through a separator which changes the pressure and temperature, thereby reducing the dissolving power of the supercritical fluid and causing the separation or fractionation of the dissolved components.

Examples of suitable supercritical fluids for the preparation of the *Tsuga* extracts include water and carbon dioxide. Carbon dioxide has a critical temperature of 31.06°C, a critical pressure of 73.83 bar, and a critical density of 0.460 $g/cm^3$, which allows the use of relatively low temperatures for the extraction process. An exemplary SCF extraction process utilising carbon dioxide as the SCF is as follows. Comminuted plant material is combined with the carbon dioxide with one or more modifiers in an extractor device. The extraction is conducted at a pressure between about 270 to about 320 bar, and a temperature of about 40°C to about 60°C. The ratio of solvent to starting plant material is typically between about 20:1 and about 80:1 by weight, for example between about 45:1 and about 60:1 by weight.

Preparation of the *Tsuga* extracts using subcritical fluids, with or without a co-solvent, is also contemplated. Examples of suitable subcritical fluids for preparation of the *Tsuga* extracts include water and carbon dioxide.

As noted above, in some embodiments, one or more co-solvents (or modifiers) are included in the supercritical fluid or subcritical fluid. Modifiers generally possess volatility between that of the supercritical or subcritical fluid and of the components being extracted, and must be miscible with the supercritical/subcritical fluid. Suitable modifiers include, for example, ethanol, methanol, propanol, acetone, ethyl acetate, methylene chloride, and the like. Water is also a suitable modifer when the supercritical/subcritical fluid is carbon dioxide. Ethanol, for example, can be used as a modifer in a ratio of 35 to 75 kg ethanol solvent per kg of plant material.

Following a typical extraction process, whether using standard pressure or sub- or supercritical fluids), the liquid fraction (the *Tsuga* extract) can be separated from the solid (insoluble) matter. Separation of the liquid and solid fractions can be achieved by one or more standard separation processes known to those skilled in the art, such as various centrifugation or filtration processes. In one embodiment of the invention, the *Tsuga* extract is separated from solid matter after the extraction by one or more filtration steps. In another embodiment, the *Tsuga* extract is separated from solid matter after the extraction by a series of filtration steps.

Once the *Tsuga* extract has been isolated, its activity can be tested directly or after being diluted in a suitable solvent, or it may be subjected to further procedures. For example, the *Tsuga* extract can be subjected to one or more additional steps to further purify or concentrate the extract. For example, the extract may be subjected to solid-liquid extraction, liquid-liquid extraction, solid-phase extraction (SPE), membrane filtration, ultrafiltration, dialysis, electrophoresis, solvent concentration, centrifugation, ultracentrifugation, liquid or gas phase chromatography (including size exclusion, affinity, and the like) with or without high pressure, lyophilization, evaporation, precipitation with various "carriers" (including PVPP, carbon, antibodies, and the like), the use of supercritical fluids (such as $CO_2$), or various combinations thereof. In one embodiment, the *Tsuga* extract is subjected to procedures to remove fatty acids or chlorophyll components that may interfere with its activity. Various procedures known in the art may be employed. In one embodiment, one or more additional partitioning steps using an organic solvent, such as hexane, heptane or ethyl acetate, are included. The liquid *Tsuga* extract can be concentrated and solubilised in an appropriate solvent prior to the one or more partitioning steps, if desired.

In one embodiment of the present invention the *Tsuga* material is subjected to an extraction process that entails contacting the solid plant material with the solvent with adequate mixing over a period of time between about 10 minutes and about 72 hours at a temperature between about 4°C and about 50°C. The liquid fraction (the *Tsuga* extract) is then separated from the solid (insoluble) matter by one or more standard processes known to those skilled in the art.

In one embodiment of the disclosure the *Tsuga* extract is prepared by extracting plant material with an alcoholic solvent alone in combination with an aqueous co-solvent for a time period between about 4 hours and about 48 hours, for example between about 4 hours and about 24 hours, at a temperature between about 4 °C to about 32°C, for example between about 4°C to about 25°C. In one embodiment of the present disclosure, the *Tsuga* extract is prepared by extracting plant material using a combination of ethanol and water as the solvent, wherein the range of ethanol:water is between about 50:50 and about 85:15, and wherein the extraction is conducted for a time period between about 4 hours and about 48 hours, for example between about 4 hours and about 24 hours, at a temperature between about 4 °C to about 32°C, for example between about 4 °C to about 25°C. In another embodiment of the disclosure, the *Tsuga* extract is prepared by extracting plant material using a combination of a glycol and water as the solvent, wherein the range of glycol:water is between about 100:0 and about 20:80, and wherein the extraction is conducted for a time period between about 4 hours and about 48 hours, for example between about 4 hours and about 24 hours, at a temperature between about 4 °C to about 32°C, for example between about 4 °C to about 25°C.

The present invention contemplates that the extraction process and any subsequent purification steps may be carried out on various scales including known large, medium and small-scale methods of preparing extracts.

In one embodiment of the invention, the *Tsuga* extract is prepared on a large-scale. For example, the *Tsuga* extract can be prepared on a commercial scale by using the extraction process employed in the initial analytical scale preparation of the extract. The small-scale extraction procedure is simply scaled-up and additional steps of quality control can be included to ensure reproducible results.

Also contemplated are modifications to the small-scale procedure as may be required during scale-up for industrial level production of the *Tsuga* extract. Such modifications include, for example, alterations to the solvent being used or to the extraction procedure employed in order to compensate for variations that occur during scale-up and render the overall procedure more amenable to industrial scale production, or more cost effective. Modifications of this type are standard in the industry and would be readily apparent to those skilled in the art.

### *Fractionation and/or isolation of active ingredients*

The present disclosure also provides for purified or semi-purified active ingredients isolated from the *Tsuga* extracts. In the context of the present invention an "active ingredient" is a compound that is present in the *Tsuga* extract and has anti-inflammatory and/or anti-microbial properties.

There are a number of techniques well known in the art for isolating active ingredients from plant extracts. For example, purification, partial purification, and/or fractionation can be performed using solid-liquid extraction, liquid-liquid extraction, solid-phase extraction (SPE), membrane filtration, ultrafiltration, dialysis, electrophoresis, solvent concentration, centrifugation, ultracentrifugation, liquid or gas phase chromatography (including size exclusion, affinity, etc.) with or without high pressure, lyophilisation, evaporation, precipitation with various "carriers" (including PVPP, carbon, antibodies, etc.), or various combinations thereof.

Thus in one embodiment of the disclosure the *Tsuga* extract is subjected to one or more of the above techniques, in a sequential fashion, in order to obtain therefrom a isolated active ingredient, or combination of active ingredients, that retains the activity of interest *(i.e.* has anti-inflammatory and/or anti-microbial activity). Isolated active ingredients can be tested for this activity according to one or more of the procedures described below. Furthermore, and where identification and/or quantification of the isolated active ingredient(s) is desired, analytical techniques including, but not limited to, NMR, GC-MS, TLC, spectrophotometry, microspray, X-ray diffraction and elemental analysis may be performed to characterise the active ingredient(s).

### TESTING THE *TSUGA* EXTRACTS

The *Tsuga* extracts in accordance with the present disclosure have anti-inflammatory and anti-microbial activity. In one embodiment of the disclosure, the extracts are additionally capable of inhibiting one or more of angiogenesis, contractile force of fibroblasts or UV-induced protease activity. These properties can be assessed using standard techniques known in the art. Exemplary techniques are provided below and in the Examples section.

### *Determination of Anti-Inflammatory Activity* in vitro

As is known in the art, keratinocytes are responsible for the immune surveillance from exogenous or intrinsic stress. Accordingly, the effect of the *Tsuga* extracts on the release of cytokines such as Interleukin-1 alpha (IL-1) as part of the inflammatory response can be assayed. This assay employs a model comprising keratinocytes in a growth medium that includes a suitable amount of the *Tsuga* extract. After an appropriate incubation period, the keratinocytes are exposed to UV light to induce an inflammatory response and are subsequently incubated for a further period of time, for example, about 24 hours. The amount of IL-1 produced by the keratinocytes in response to the UV treatment can be assessed, for example, by a specific ELISA that quantitates IL-1. The amount of IL-1 can be compared to an untreated control and/or to a control treated with a compound known to inhibit inflammation.

The anti-inflammatory effects of the *Tsuga* extracts can also be tested in an *in vitro* psoriasis model. As is known in the art, keratinocytes undergo differentiation in the different layers of the epidermis. In the process, several markers are expressed that reflected the level of differentiation. Accordingly, the effect of the *Tsuga* extracts on the expression of such markers on psoriatic-like induced keratinocytes can be assayed. This assay employs a model comprising keratinocytes in a rich serum growth media that includes a suitable amount of the *Tsuga* extract. After an appropriate incubation period, the keratinocytes are fixed with formaldehyde and assayed for different differentiation markers such as involucrin, Transglutaminase, cytokeratin-10 and 16 and/or SKALP, for example, using indirect immunofluorescence. The amount of each marker can be evaluated qualitatively or quantitatively. The level of markers induced in keratinocytes treated with the *Tsuga* extract can be compared to an untreated control and/or to a control treated with a compound known to treat psoriasis, such as dithranol.

### Determination of Anti-Inflammatory Activity *in vivo*

The ability of the plant extracts to reduce inflammation in the skin can be assessed in human volunteers using standard techniques. For example, the Minimal Erythema Dose (MED) test measures the minimum UV dose required to produce a distinct reaction in the skin in the form of redness, which appears 24 hours after exposure. The MED of all volunteers must be measured prior to any treatment that attempts to measure the skin response to UV induced erythema.

In general, at Day D0, the skin color for each volunteer is measured in the test zone (usually the forearm) using a Mexameter® to approximate the MED of the individual volunteer. Actinic erythema is induced using a UV simulator to the selected zone by applying a series of 5 consecutive increasing doses of irradiation to two sites on the skin - the first, a non-treated site on one forearm and the second, a site treated with the *Tsuga* extract (typically formulated into a cream) on the other forearm. The skin response on each forearm is then assessed visually 24 hrs later (Day D1) to calculate the individual MED (the lowest dose to which a distinct reaction is observed).

Other tests, such as the sodium lauryl sulphate (SLS) patch test and the Mantoux test, which involves the injection of tuberculin, are known in the art and may be employed to test the anti-inflammatory effects of the *Tsuga* extracts. Various noninvasive measuring methods can be used to evaluate the skin before and after exposure to the applied irritants, for example, measurement of transepidermal water loss by an evaporimeter, measurement of electrical conductance by a hydrometer, measurement of skin blood flow by laser Doppler flowmetry, measurement of skin colour using a colorimeter and/or measurement of skin thickness by ultrasound A-scan (see, for example, Agner, T., (1992) Acta Derm Venereol Suppl (Stockh). 173:1-26, and De Fine Olivarious, F., et al. (1993) Br J Dermatl. 129:554-557).

Attenuation of psoriasis lesions can be evaluated by conducting tests using a panel of human volunteers with psoriatic plaques. The test typically involves application of the *Tsuga* extract (typically formulated into a cream) to the affected areas of the patients' skin on a regular basis, such as once or twice a day, over a period of several weeks. The effect of the *Tsuga* extract on the psoriasis plaques can be evaluated by visually inspecting the treated plaques and assessing the characteristic or characteristics being investigated, for example, decrease of redness, lesions and/or desquamation. Other aspects, such as decrease in itchiness can also be investigated.

### Determination of Anti-*Microbial* Activity *in vitro*

The ability of the *Tsuga* extracts to inhibit the growth of microbial cells can be assessed using standard *in vitro* methods known in the art. In general, these methods involve contacting a culture of the microbial cells with various concentrations of the extract and monitoring the growth of the cell culture relative to an untreated control culture. A second control culture comprising cells contacted with a known anti-microbial agent may also be included in such tests, if desired. An exemplary test for determining the ability of the *Tsuga* extract to inhibit a variety of bacterial species is provided in the Examples (see Example XI).

The ability of the *Tsuga* extracts to inhibit the growth of bacterial cells can also be determined by measurement of the minimum inhibitory concentration (MIC). The MIC is defined as the lowest concentration that inhibits growth of the organism to a pre-determined extent. For example, a $MIC_{100}$ value is defined as the lowest concentration that completely inhibits growth of the organism, whereas a $MIC_{90}$ value is defined as the lowest concentration that inhibits growth by 90% and a $MIC_{50}$ value is defined as the lowest concentration that inhibits growth by 50%. MIC values are sometimes expressed as ranges, for example, the $MIC_{100}$ for a compound may be expressed as the concentration at which no growth is observed or as a range between the concentration at which no growth is observed and the concentration of the dilution which immediately follows.

Typically, anti-bacterial MICs are measured using a broth macro- or microdilution assay (see Amsterdam, D. (1996) "Susceptibility testing of antimicrobials in liquid media," pp.52-111. In Loman, V., ed. Antibiotics in Laboratory Medicine, 4th ed. Williams and Wilkins, Baltimore, MD). A standardised anti-bacterial susceptibility test is provided by the National Committee for Clinical Laboratory Standards (NCCLS) as NCCLS, 2000; document M7-A58.

In the classical broth microdilution method, the *Tsuga* extract would be diluted in culture medium in a sterile, covered 96-well microtiter plate. An overnight culture of a single bacterial colony is diluted in sterile medium such that, after inoculation, each well in the microtiter plate contains an appropriate number of colony forming units (CFU)/ml (typically, approximately $5 \times 10^5$ CFU/ml). Culture medium only (containing no bacteria) is also included as a negative control for each plate and known antibiotics are often included as positive controls. The inoculated microtiter plate is subsequently incubated at an appropriate temperature (for example, 35°C - 37°C for 16-48 hours). The turbidity of each well is then determined by visual inspection and/or by measuring the absorbance, or optical density (OD), at 595nm or 600nm using a microplate reader and is used as an indication of the extent of bacterial growth.

## In vitro *Cellular Activity*

The ability of the *Tsuga* extracts to affect one or more of angiogenesis, contractile force of fibroblasts or UV-induced protease activity can be assessed *in vitro* using one, or a combination, of standard techniques known in the art.

For example, inhibition of angiogenesis can be assessed using the cord formation assay. In this assay, endothelial cells with or without the *Tsuga* extract are plated onto Matrigel® and incubated under appropriate conditions. After a suitable period of time, (for example, between 18 and 24 hours), the evaluation of angiogenesis is assessed by visual inspection to determine whether the cells have formed into cords.

Various cell lines can be used in cord formation assays. Examples of suitable endothelial cell lines include, but are not limited to, human umbilical vein endothelial cells (HUVECs), bovine aortic endothelial cells (BAECs), human coronary artery endothelial cells (HCAECs), bovine adrenal gland capillary endothelial cells (BCE) and vascular smooth muscle cells. HUVECs can be isolated from umbilical cords using standard methods (see, for example, Jaffe et al. (1973) J. Clin. Invert. 52(11): 2745-56), or they can be obtained from the ATCC or various commercial sources, as can other suitable endothelial cell lines.

The effect of the *Tsuga* extracts on the tractional forces generated by fibroblasts can be assayed, for example, using a model comprising fibroblasts embedded in a collagen matrix to create a derm-like environment. Such a model can be prepared by adding fibroblasts to a solution of collagen I in medium and then allowing the collagen to polymerize to form a gel. After an appropriate incubation period, the derm-like gel is treated with the *Tsuga* extract and the amount of contraction measured over a period of time, for example, several days. The amount of contraction can be assessed for example, by digitally photographing the gel at various time points and calculating the gel area using appropriate software. The amount of contraction can be compared to untreated control gels and/or gels treated with a compound known to affect fibroblast tractional forces.

UV-induced extracellular protease activity can be assessed by irradiating cultures of skin cells with UVA light and then treating the irradiated cells with the *Tsuga* extract. Alternatively, the extract can be added to the cells prior to irradiation to assess the prophylactic effect of the extract. After a suitable period of incubation in an appropriate medium, supernatants can be removed from the cells and assayed for proteolytic activity, such as MMP-9 activity as described below. Results can be compared to untreated cells and/or cells treated with a compound known to affect UV-induced protease activity.

Skin cells suitable for use in the above assay include human dermal fibroblasts, keratinocytes, melanocytes, Langerhans cells, cells of the hair follicle and cells of the immune system which produce proteases, including leukocytes, macrophages and lymphocytes.

## In vivo *Cellular Activity*

The *Tsuga* extracts may undergo additional testing on human volunteers to assess their ability to exert the desired dermatological effect(s).

For example, the effect of the extracts on skin changes, such as wrinkling and/or sagging, reddening, formation of lesions, abnormal pigmentation and the like, can be assessed by visual examination. For example, the effect of the *Tsuga* extract on the skin can be evaluated by formulating the extract such that it is suitable for external application to the skin and subsequently sensory tests can be conducted on the formulation using a panel of human volunteers. A sensory test typically involves application of the formulation to the skin of the panelists on a regular basis, such as once or twice a day, over a period of several weeks. The effect of the formulation on the skin can be evaluated by inspecting the skin of the panelists and assessing visually the skin characteristic or characteristics being investigated, for example, the tenseness and gloss of the skin, the appearance of existing wrinkles and sags, reddening, lesions and/or abnormal pigmentation. Attenuation of Crow's feet can be determined, for example, using a computerized digital image to obtain a skin's topography before and after treatment in human volunteers. Erythema in skin samples can be determined, for example, using commercially available chromameter.

The plant extracts may also undergo one or more safety, stability and/or bioavailability test prior to testing on human volunteers, for example, the Human Repeat Insult Patch Test (HRIPT).

*Other Tests*

*Determination of MMP-9 Inhibitory Activity*

A variety of methods and techniques for measuring the ability of the *Tsuga* extracts to inhibit the activity of MMP-9 either qualitatively or quantitatively are known in the art. For example, there are currently several assays to measure the activity of MMPs (including MMP-9) (for a review of these methods, see Murphy and Crabbe, In Barrett (ed.) Methods in Enzymology. Proteolytic Enzymes: Aspartic Acid and Metallopeptidases, New York: Academic Press, 1995, 248: 470), including the gelatineolytic assay (which is based on the degradation of radio-labelled type I collagen), the zymography assay (which is based on the presence of negatively-stained bands following electrophoresis through substrate-impregnated SDS polyacrylamide gels) and a microtitre plate assay developed by Pacmen et al., (Biochem. Pharm. (1996) 52:105-111).

Other methods include those that employ auto-quenched fluorogenic substrates. Many fluorogenic substrates have been designed for quantification of the activity of MMPs through fluorescent level variation measuring (reviewed by Nagase and Fields (1996) Biopolymers 40: 399-416). Another method of measuring MMP-9 activity makes use of the fluorescent activated substrate conversion (FASC) assay described in Canadian Patent No. 2,189,486 and in St-Pierre et al. ((1996) Cytometry 25: 374-380).

Alternatively, the ability of the *Tsuga* extracts to inhibit the activity of MMP-9 can be evaluated using cultures of cells that secrete MMPs. In this case a cell culture is in contact with an appropriate amount of the extract. After an appropriate period of time, the cells are extracted, centrifuged and the proteolytic activity in the supernatant is measured. This method is useful in determining the ability of an extract to inhibit MMP-9 secreted by a particular cell line or combination of cell lines. For example, assays can be conducted with cell lines derived from mammalian skin, such as keratinocytes or fibroblasts.

In vivo *Testing of MMP-9 Inhibition*

The ability of the *Tsuga* extracts to inhibit MMP-9 activity *in vivo* may be assessed using various standard techniques. For example, the ability of the *Tsuga* extracts to inhibit protease activity can be determined in animal models or human volunteers. An example of a suitable animal model would be a skh-1 mouse or nude mouse or rat that is treated with a *Tsuga* extract and then exposed to UV radiation (see, Nishimori et al. (2001) J. Invert. Dermatol. 117:1458-1463). UV radiation is known to increase the level of activity of certain MMPs (see, for example, U.S. Patent No. 6,130,254). Skin biopsies are taken from the animal and the amount of MMP-9 activity in the biopsied sample can be measured using standard techniques as an indication of the inhibitory activity of the test extract.

Human trials may also be used to evaluate the ability of the *Tsuga* extract to inhibit MMP-9 activity in the skin. For example, skin biopsies can be taken from adult volunteers exposed to UV radiation and treated prior to or after UV exposure with an extract. The biopsy samples can be assessed for MMP-9 activity and compared to an appropriate control (for example, skin biopsies from individuals treated with a control compound or untreated individuals). Alternatively, as an age-related increase in the relative activities of MMP-1, MMP-2 and MMP-9 has been demonstrated (see, for example, U.S. Patent Application No. 20010053347), elderly individuals (for example, those over 80 years of age) could be used as volunteers for the trials without the requirement for UV exposure.

In order to assess the protease activity in skin biopsies, the samples are typically flash frozen, mechanically ground and/or homogenised. After centrifugation, the supernatants are isolated and used to assess MMP-9 activity in assays such as those outlined above.

*Safely Testing*

In addition to the above tests, the *Tsuga* extracts may be submitted to other standard tests to evaluate safety, cytotoxicity, stability, bioavailability and the like. Exemplary tests to determine the cytotoxicity of the extracts and their potential to induce cytokine release are described herein (see Examples III and VI).

The ability of the *Tsuga* extract to penetrate the skin can be assessed, for example, by *in vitro* release tests (see, for example, the U.S. Center for Drug Evaluation and Research guidance document entitled "*Guidance for Industry. Nonsterile Semisolid Dosage Forms. Scale-up and postapproval changes: in vitro release testing and in vivo bioequiv-*

*alence documentation*"). Typically, such testing is conducted using an open chamber diffusion cell, such as a Franz cell, fitted with an appropriate membrane. The test extract is placed on the upper side of the membrane and kept occluded to prevent solvent evaporation and compositional changes. A receptor fluid, such as aqueous buffer or hydro-alcoholic medium, is placed on the other side of the membrane in a receptor cell. Diffusion of the active component across the membrane is monitored by assay of sequentially collected samples of the receptor fluid. For the *Tsuga* extracts in accordance with the invention, the assay could comprise, for example, testing the ability of the collected sample to inhibit MMP-9 activity. The membrane can be a synthetic membrane, for example polysulphone, cellulose acetate or nitrate, or polytetrafluoroethylene, or it can be a skin sample, such as a sample taken from a cadaver.

Other tests are known in the art (for example, see U.S. Pharmacopoeia XXII (1990)) and are suitable for testing the stability and/or safety of the *Tsuga* extracts.

As will be readily apparent to one skilled in the art, a selected extract may need to meet certain criteria in order to meet regulatory requirements for human use. Conducting tests such as those described above, therefore, allows the suitability of an extract for human use to be assessed.

## DERMATOLOGICAL FORMULATIONS

The present invention further provides for formulations comprising a butylene glycol:water 50:50 (v/v) extract from needles, twigs or small branches of Tsuga canadensis suitable for dermatological, including cosmetic, applications. The formulations can optionally comprise other active agents, such as therapeutic or cosmetic agents and/or other plant extracts. The formulations are prepared by standard techniques such that they have acceptable toxicity and stability.

The formulations are prepared by mixing a suitable amount of the *Tsuga* extract with a physiologically acceptable carrier or diluent. In one embodiment of the invention, the formulation comprises only the *Tsuga* extract and a diluent or carrier. In another embodiment of the invention, the formulation comprises the *Tsuga* extract and one or more thickener, excipient, binder or the like, and optionally one or more other active agent.

Suitable amounts of the *Tsuga* extract for incorporation into the dermatological formulation are generally in the range of between about 0.1% (v/v) and about 20% (v/v). In one embodiment, the dermatological formulation comprises between about 0.1% (v/v) and about 18% (v/v) of the *Tsuga* extract. In another embodiment, the dermatological formulation comprises between about 0.1% (v/v) and about 16% (v/v) of the *Tsuga* extract. In another embodiment, the dermatological formulation comprises between about 0.1% (v/v) and about 15% (v/v) of the *Tsuga* extract. In other embodiments, the dermatological formulation comprises between about 0.1% (v/v) and about 14% (v/v), between about 0.1% (v/v) and about 12% (v/v), between about 0.1% (v/v) and about 10% (v/v), between about 0.1% (v/v) and about 8% (v/v), and between about 0.1% (v/v) and about 6% (v/v) of the *Tsuga* extract.

In other embodiments, the dermatological formulation comprises between about 0.2% (v/v) and about 20% (v/v), between about 0.4% (v/v) and about 20% (v/v), between about 0.5% (v/v) and about 20% (v/v), between about 0.5% (v/v) and about 18% (v/v), between about 0.6% (v/v) and about 15% (v/v), between about 0.8% (v/v) and about 12% (v/v), and between about 1.0% (v/v) and about 10% (v/v) of the *Tsuga* extract.

The amount of *Tsuga* extract for incorporation into the dermatological formulation can also be defined as % by weight. For example, suitable amounts of the *Tsuga* extract for incorporation into the dermatological formulation are generally in the range of between about 0.1% and about 20% by weight. In various embodiments of the invention, the dermatological formulation comprises between about 0.1% and about 18% by weight, between about 0.1% and about 16% by weight, between about 0.1% and about 15% by weight, between about 0.1% and about 14% by weight, between about 0.1% and about 12% by weight, between about 0.1% and about 10% by weight, between about 0.1% and about 8% by weight, between about 0.1% and about 6% by weight, between about 0.2% and about 20% by weight, between about 0.4% and about 20% by weight, between about 0.5% and about 20% by weight, between about 0.5% and about 18% by weight, between about 0.6% and about 15% by weight, between about 0.8% and about 12% by weight, and between about 1.0% and about 10% by weight of the *Tsuga* extract.

In one embodiment of the present invention, the dermatological formulations comprising the Tsuga canadensis extract are formulated for topical administration. Such formulations may be presented as, for example, aerosol sprays, powders, sticks, granules, creams, liquid creams, mousses, pastes, gels, lotions, ointments, on sponges or cotton applicators, or as a solution or a suspension in an aqueous liquid, a nonaqueous liquid, an oil-in-water emulsion, or a water-in-oil liquid emulsion.

Various physiologically acceptable carriers are known in the art. Examples of such carriers include, but are not limited to, hydroxypropyl cellulose, starch (corn, potato, rice, wheat, quinoa), pregelatinized starch, gelatine, sucrose, acacia, alginic acid, sodium alginate, guar gum, ethyl cellulose, carboxymethylcellulose sodium, carboxymethylcellulose calcium, polyvinylpyrrolidone, methylcellulose, hydroxypropyl methylcellulose, microcrystalline cellulose, polyethylene glycol, powdered cellulose, glucose, croscarmellose sodium, crospovidone, polacrilin potassium, sodium starch glycolate, tragacanth, calcium carbonate, dibasic calcium phosphate, tribasic calcium phosphate, kaolin, mannitol, talc, cellulose acetate phthalate, polyethylene phthalate, shellac, titanium dioxide, carnauba wax, microcrystalline wax, calcium stearate, magnesium stearate, castor oil, mineral oil, light mineral oil, glycerine, sorbitol, mannitol, stearic acid, sodium lauryl sulfate, hydrogenated vegetable oil (for example, peanut, cottonseed, sunflower, sesame, olive, corn, soybean), zinc stearate, ethyl oleate, ethyl laurate, agar, calcium silicate, magnesium silicate, silicon dioxide, colloidal silicon dioxide, calcium chloride, calcium sulfate, silica gel, castor oil, diethyl phthalate, glyercine, mono- and di-acetylated monoglycerides, propylene glycol, triacetin, alamic acid, aluminum monostearate, bentonite, bentonite magma, carbomer 934, carboxymethylcellulose sodium 12, carrageenan, hydroxyethyl cellulose, magnesium aluminum silicate, pectin, polyvinyl alcohol, povidine, sodium alginate, tragacanth, xanthan gum, silicones and various combinations thereof.

Exemplary thickeners are cross-linked polyacrylate materials available under the trademark Carbopol™ (B. F. Goodrich Company), xanthan gum, carrageenan, gelatine, karaya, pectin and locust bean gum. Under certain circumstances the thickening function may be accomplished by a moisturiser component of the formulation. For instance, silicone gums and esters such as glycerol stearate have dual functionality. A thickener will usually be present in amounts from 0.1 to 20% by weight of the formulation.

The formulations can optionally include one or more moisturising agents, *i.e.* an agent that facilitates hydration of the skin by inhibiting or preventing loss of water from the skin, that absorbs water from the atmosphere and hydrates the skin, and/or that enhances the skin's ability to absorb water directly from the atmosphere. Moisturising agents generally minimise or prevent the skin from drying and cracking. Moisturisers, when used, are typically present in an amount from about 0.01 to 20% by weight of the formulation.

Examples of moisturising agents include, but are not limited to, 2-hydroxyacetic acid (glycolic acid); 2-hydroxypropanoic acid (lactic acid); 2-methyl 2-hydroxypropanoic acid; 2-hydroxybutanoic acid; phenyl 2-hydroxyacetic acid; phenyl 2-methyl 2-hydroxyacetic acid; 3-phenyl 2-hydroxyacetic acid; 2,3-dihydroxypropanoic acid; 2,3,4-trihydroxybutanoic acid; 2,3,4,5,6-pentahydroxyhexanoic acid; 2-hydroxydodecanoic acid; 2,3,4,5-tetrahydroxypentanoic acid; 2,3,4,5,6,7-hexahydroxyheptanoic acid; diphenyl 2-hydroxyacetic acid; 4-hydroxymandelic acid; 4-chloromandelic acid; 3-hydroxybutanoic acid; 4-hydroxybutanoic acid; 2-bydroxyhexanoic acid; 5-hydroxydodecanoic acid; 12-hydroxydodecanoic acid; 10-hydroxydecanoic acid; 16-hydroxyhexadecanoic acid; 2-hydroxy-3-methylbutanoic acid; 2-hydroxy-4-methylpentanoic acid; 3-hydroxy-4-methoxymandelic acid; 4-hydroxy-3-methoxymandelic acid; 2-hydroxy-2-methylbutanoic acid; 3-(2-hydroxyphenyl) lactic acid; 3-(4-hydroxyphenyl) lactic acid; hexahydromandelic acid; 3-hydroxy-3-methylpentanoic acid; 4-hydroxydecanoic acid; 5-hydroxydecanoic acid; aleuritic acid; 2-hydroxypropanedioic acid; 2-hydroxybutanedioic acid; tannic acid; salicylic acid; erythraric acid; threaric acid; arabiraric acid; ribaric acid; xylaric acid; lyxaric acid; glucaric acid; galactaric acid; mannaric acid; gularic acid; allaric acid; altraric acid; idaric acid; talaric acid; 2-hydroxy-2-methylbutanedioic acid; citric acid, isocitric acid, agaricic acid, quinic acid, glucoronic acid, glucoronolactone, galactoronic acid, galactoronolactone, uronic acids, uronolactones, ascorbic acid, dihydroascorbic acid, dihydroxytartaric acid, tropic acid, ribonolactone, gluconolactone, galactonolactone, gulonolactone, mannonolactone, citramalic acid; pyruvic acid, hydroxypyruvic acid, hydroxypyruvic acid phosphate and esters thereof; methyl pyruvate, ethyl pyruvate, propyl pyruvate, isopropyl pyruvate; phenyl pyruvic acid and esters thereof; methyl phenyl pyruvate, ethyl phenyl pyruvate, propyl phenyl pyruvate; formyl formic acid and esters thereof; methyl formyl formate, ethyl formyl formate, propyl formyl formate; benzoyl formic acid and esters thereof; methyl benzoyl formate; ethyl benzoyl formate; propyl benzoyl formate; 4-hydroxybenzoyl formic acid and esters thereof; 4-hydroxyphenyl pyruvic acid and esters thereof; and 2-hydroxyphenyl pyruvic acid and esters thereof. It should be understood that one or more derivatives of the above compounds, including esters or lactones or pharmaceutically acceptable salts thereof, may also be used.

Further examples of moisturising agents include, but are not limited to, beeswax, shea butter, ceramide, borage oil (linoleic acid), tocopherol linoleate, dimethicone, glycerine, hyaluronic acid, sodium peroxylinecarbolic acid (sodium PCA), wheat protein (such as laurdimonium hydroxypropyl hydrolyzed wheat protein), primrose oil, flax seed oil and mixtures thereof.

As noted above, the dermatological compositions may optionally include one or more other active agents and/or plant extracts that are intended to impart additional beneficial properties to the formulation. For example, the dermatological formulation can also include one or more additional anti-inflammatory components which facilitate inhibition or suppression of inflammation on or in the skin or in adjacent bodily tissues and thereby help to reduce redness and swelling of the skin. Examples of suitable anti-inflammatory components include vitamin E and derivatives thereof, zinc, allantoin, glycyrrhetic acid, azulene, mefenamic acid, phenylbutazone, indometacin, ibuprofen, ketoprofen, aminocaproic acid, hydrocortisone, panthenol and derivatives and salts thereof, zinc oxide, salicylic acid and diclofenac sodium. The anti-inflammatory component, when used, can be incorporated into the formulations of the present invention in an amount between about 0.001 to about 5 % by weight.

Other examples of skin benefit ingredients that may be included in the formulations in various embodiments of the invention include, but are not limited to, anti-oxidants, retinols and retinol derivatives (including pro-retinol), alpha-hydroxy acids (AHAs), skin whitening agents and other anti-ageing ingredients. Examples of retinol and retinol derivatives include, but are not limited to, retinol, retinoic acid (tretinoin), isotretinoin and retinaldehyde. Examples of AHAs include, but are not limited to, lactic acid and glycolic acid. Examples of skin whitening agents include, but are not limited to, kojic acid, kojic dipalmitate, hydroquinone, arbutins (such as alpha-arbutin, beta-arbutin and deoxy-arbutin) and azelaic acid.

Tretenoin is also known to have slight skin whitening properties and may be used in combination with another skin whitening agent.

The formulations may also optionally comprise one or more of a sunscreen, a sunblock, essential fatty acids, a cell activator, a blood-circulation promoter, a tanning agent, proteins, peptides and polysaccharides.

Azoles, such as climbazole, bifonazole, clotrimazole, ketoconazole, miconazole, econazole, itraconazole, fluconazole, terconazole, butoconazole, sulconazole, lionazole and mixtures thereof, may also optionally be included in the formulations.

In one embodiment of the invention, the dermatological formulation comprises one or more additional anti-ageing ingredients. In another embodiment, the dermatological formulation comprises retinol or a retinol derivative. In another embodiment, the dermatological formulation comprises an AHA. In another embodiment, the dermatological formulation comprises retinol or a retinol derivative or an AHA in a higher than standard amount. As is known in the art, retinol is generally added to skin care formulations in amounts between about 0.1% and about 0.5% by weight, and AHAs are generally added in amounts between about 1% and about 6% by weight. Kojic acid or hydroquinone are generally included in dermatological compositions in amounts up to 4% by weight. Accordingly, in the context of the present invention a "higher than standard amount" of retinol is considered to be 0.5% by weight or greater, and a "higher than standard amount" of an AHA is considered to be 5% by weight or greater.

In one embodiment, the formulation comprises 0.6% by weight or higher of retinol or a retinol derivative. In other embodiments, the formulation comprises 0.7% by weight or higher, 0.8% by weight or higher, 0.9% by weight or higher, or 1.0% by weight or higher, of retinol or a retinol derivative. In one embodiment, the formulation comprises 6% by weight or higher of an AHA. In other embodiments, the formulation comprises 6.5% by weight or higher, 7.0% by weight or higher, 7.2% by weight or higher, 7.5% by weight or higher, 7.8% by weight or higher, or 8.0% by weight or higher, of an AHA. In one embodiment, the formulation comprises 4% by weight or higher of a whitening agent, such as kojic acid, kojic dipalmitate or hydroquinone. In other embodiments, the formulation comprises 4.5% by weight or higher, 5.0% by weight or higher, 5.2% by weight or higher, 5.5% by weight or higher, 6.0 % by weight or higher of a whitening agent, such as kojic acid, kojic dipalmitate or hydroquinone.

In another embodiment, the dermatological formulation comprises one or more other plant extracts.

Other adjunct minor components can also optionally be incorporated into the dermatological formulations, for example, colouring agents, opacifiers, perfumes and preservatives (for example, imidazolidinyl urea, dimethyl imidazolidinone or diazolidinyl urea). Amounts of these materials can range from 0.001% up to 20% by weight of the formulation.

The dermatological formulations intended for topical application can be packaged in a suitable container to suit the viscosity and intended use. For example, a lotion, fluid cream, foam or mousse can be packaged in a bottle or a roll-ball applicator, a capsule, a propellant-driven aerosol device or a container fitted with a pump suitable for finger operation. When the composition is a cream or paste, it can simply be stored in a non-deformable bottle or squeeze container, such as a tube or a lidded jar.

USE

The present invention provides for the use of the extracts in the treatment of microbial infection associated with acne.

The present disclosure also provides for the use of the *Tsuga* extracts and formulations comprising same to ameliorate inflammation, irritation and/or infection in a subject. In one embodiment, the disclosure provides for the use of to ameliorate the effects of inflammation, irritation or infection, for example, by promoting the formation of scar tissue and/or promoting healing of wounds caused by the inflammation, irritation or infection.

For example, the *Tsuga* extracts of the disclosure and dermatological formulations comprising the *Tsuga* extracts are suitable for a variety of applications in the dermatological field. For example, in one embodiment, the disclosure provides for the use of the *Tsuga* extracts and formulations for the treatment of dermatological conditions and, in particular, dermatological conditions with an associated inflammatory component or infection. For example, the extracts and formulations can be used to treat psoriasis, rosacea, acne, dermatitis (for example, atopic dermatitis, professional dermatitis and/or seborrheic dermatitis), erythema, eczema, pruritis, lichen simplex, photodermatosis (for example, polymorphous light eruption and actinic prurigo), prurigo nodulans, sunburn, hives, and rashes (including diaper rash). The extracts and formulations can also be used for treating obesity-related skin problems (for example, inflammation, redness, erythema, rashes and/or bacterial infections caused by skin folds) and for combating the irritant or inflammatory effects of, or infection due to, cosmetic or surgical skin procedures, such as peeling, exfoliation, laser treatments, hair removal or plastic surgery.

In one embodiment, the invention provides for the use of the Tsuga canadensis extracts and formulations comprising same for the treatment of acne. In another embodiment, the disclosure provides for the use of the *Tsuga* extracts and formulations comprising same for the treatment of erythema. Erythema can be caused by, for example, infection, massage, electrical treatment, acne medication, allergies, exercise, solar radiation (such as sunburn), cutaneous radiation syndrome, hair removal (for example, plucking, waxing, laser removal), laser treatments or radiotherapy.

In another embodiment, the disclosure provides for the use of the *Tsuga* extracts in formulations comprising skin treatment compounds that can exert irritant or inflammatory effects on the skin of the user, for example, retinol, retinol derivatives, AHAs and/or skin whitening agents, in order to ameliorate the irritant/inflammatory effects of these compounds. In another embodiment, the disclosure provides for the use of the *Tsuga* extracts in dermatological formulations for sensitive skins. In another embodiment, the disclosure provides for the use of the *Tsuga* extracts in dermatological formulations for ameliorating the irritant/inflammatory effects of environmental factors, for example in "after sun" formulations, and/or of cosmetic skin procedures, for example in "after shave" formulations.

The *Tsuga* extracts can also be incorporated into dermatological formulations in order to provide a preservative effect due to their anti-microbial activity.

One embodiment of the present disclosure provides for the use of the *Tsuga* extract as an anti-inflammatory agent. Another embodiment of the disclosure provides for the use of the *Tsuga* extract as an anti-microbial agent. Another embodiment of the disclosure provides for the use of the *Tsuga* extract to ameliorate the inflammatory and/or irritation symptoms of a dermatological condition.

Another embodiment of the present disclosure provides for the use of the *Tsuga* extract in an anti-ageing product. An anti-ageing product is a product intended for use to attenuate skin ageing that occurs due to intrinsic or extrinsic factors. The anti-ageing product may comprise other anti-ageing compounds in addition to the *Tsuga* extract. Skin ageing phenomena that can be treated with anti-ageing products include, for example, skin thinning, fine and coarse skin wrinkling, sagging, loss of elasticity, and the like.

Another embodiment of the disclosure provides for the use of the *Tsuga* extract and formulations comprising the *Tsuga* extract to promote the formation of scar tissue. A further embodiment provides for the use of the *Tsuga* extract and formulations comprising the *Tsuga* extract to promote wound healing.

To gain a better understanding of the invention described herein, the following examples are set forth. It should be understood that these examples are for illustrative purposes only.

## EXAMPLES

### EXAMPLE I: *Preparation of Extracts from* Tsuga canadensis

#### *Analytical Scale Preparation*

In general, five grams of the dried plant material to be extracted was placed in a beaker and a sufficient amount of solvent was added to allow moderate agitation with a stirring bar. The solvents used in this Example were: butylene glycol (100%), butylene glycol/water (50/50, v/v), butylene glycol/water (20/80, v/v); ethanol (100%), ethanol/water (85/15, v/v), ethanol/water (50/50, v/v); water (100%); 1,3-propanediol (100%); 1,3-propanediol/water (50/50, v/v) and 1,3-propanediol/water (20/80, v/v).

Several different extraction times were employed for each solvent: after mixing for periods of 4 to 24 hours at room temperature, the suspension was filtered through a 60mesh filter and then filtered through 40, 20, 11 and 1.2 micron paper filter consecutively. For the filtered butylene glycol mixtures, the solvent was then evaporated at 120°C and the residual matter was weighed to determine the yield of extraction at each time point. For the filtered ethanol mixtures, the solvent was removed under reduced pressure at a temperature of less than 45°C in order to determine the yield of extraction at each time point.

The above protocol is suitable for the preparation of extracts that are to be employed in dermatological formulations. Glycol extracts such as butylenes glycol or 1,3-propanediol extracts, for example, can be included directly into formulations intended for topical application. Ethanol extracts may undergo one or more additional steps, such as $CO_2$ extraction, prior to incorporation into formulations intended for topical application.

The following is an example of a specific protocol that was employed to prepare a *Tsuga canadensis* extract.

To 5 g of dried needles and small branches of *Tsuga canadensis* lot 20252, 100ml of a 50:50 butylene glycol:water was added. The mixture was thoroughly stirred and macerated for 4 hours at room temperature. The resulting mix was filtered through 40μ to 1.2μ filters to obtain a suitable extract for further analysis.

The Tsuga canadensis extracts 20252-PLA; 20156A; 207-20156A and 20010 1NG13B tested in the following examples are all extracts prepared from needles and small branches of Tsuga canadensis using 50:50 butylene glycol:water as the extraction solvent following the protocol outlined above. The extracts differed in the individual Tsuga canadensis tree that the plant material came from and in the time that the plant material was harvested.

The Tsuga canadensis extract 20376 1NG23ZB also tested in the following examples are extracts prepared from needles of *Tsuga canadensis* using 50:50 1.3-propanediol:water as the extraction solvent following the protocol outlined above.

*Large Scale Preparation*

Analysis of the results from the analytical scale preparation allows for the selection of appropriate plant materials for the large-scale extraction. The selection includes a decision regarding part of the *Tsuga* tree and quantity of dried material needed to obtain a sufficient yield on a large scale. The selection also involves a choice of solvent system for an active extract.

The extraction protocol is essentially the same as the procedure for the analytical preparation except for the filtration process. The dried and pulverized material (2-3 Kg for large scale) is extracted repeatedly (maceration / percolation) with solvent (3 x 2 - 20 l) at room temperature for 24-72 h, based on the analytical scale yield of extraction results. The resulting extract is then passed through a 60 mesh filter and then may be filtered through 10 to 100kDa ultrafiltration column or on a 0.1 to 1μ ceramic column.

Non-limiting examples of large scale extraction preparations of extracts from *Tsuga canadensis* are provided below.

A. To 3kg of dried needles and small branches from *Tsuga canadensis* lot 20252, 60L of a 50:50 Butylene glycol:water was added. The mixture was thoroughly stirred and macerated for 24 hours. The resulting mix was filtered through a 65mesh filter and then filtered on a 1μ ceramic column to obtain an extract suitable for further analysis.

B. To 3kg of dried needles and small branches from *Tsuga canadensis* lot 20252, 60L of a 50:50 Butylene glycol:water or 1,3-propanediol:water was added. The mixture was thoroughly stirred and macerated for 24 hours, then decanted for another 18-24hours. The resulting mix was filtered through a 65mesh filter and then filtered by ultrafiltration at 100kDa to obtain an extract suitable for further analysis.

## EXAMPLE II: In vitro *MMP-9 Inhibition Assays*

MMP-9 was purified from natural sources (human cell lines, THP-1 (ATCC, Manassas, VA)) as described in literature and based on protocols found in I.M. Clark: *"matrix metalloproteinases protocols",* Humana Press (2001). Proteolytic activity of the protease was evaluated with the assay based on the cleavage of auto-quenched peptide substrate : (MCA-Pro-Leu-Gly-Leu-Dpa-Ala-Arg-NH$_2$·TUFA [Dpa = N-3-(2,4-dinitrophenyl)-L-2,3-diaminopropionyl]). In the intact peptide, Dpa or DNP quenches the MCA fluorescence. Cleavage of the peptide causes release of the fluorescent MCA group which was then quantified on a fluorometer (Gemini XS, Molecular Devices, Sunnyvale, CA). The assay was performed in TNCZ assay buffer (20mM Tris-HCl; NaCl 150mM; CaCl$_2$ 5mM; ZnCl$_2$ 0.5mM; pH 7.5) with purified human MMP-9 (I.M. Clark: Matrix metalloproteinases protocols, Humana Press (2001)). The substrate, primarily dissolved in DMSO was then redissolved in TNCZ buffer for the assay.

In a typical fluorescent assay, 10 μl of purified enzyme at concentrations previously mentioned for the enzymatic assay, 5 μL of dissolved fluorogenic peptide and 40μL of the *Tsuga* extract to be tested were mixed in a final volume of 75 μl, completed with TNCZ. All assays were performed in 96 well plates and the reaction was started by the addition of substrate. Assays were measured (excitation 325 nm, emission 392 nm) for 20, 40 and 60 minutes. Activity and inhibition values were determined from the increase in fluorescence.

The inhibition is reported as percentage (%) of inhibition of substrate degradation as compared with substrate degradation in the absence of the *Tsuga* extract. Percentage inhibition was calculated according to the formula:

$$\text{Percentage (\%) inhibition} = [E_A - E_B / E_A] \times 100$$

wherein $E_A$ is the protease activity in the absence of the plant extract and $E_B$ is the protease activity in the presence of the extract.

Following the above protocol, *Tsuga canadensis* extract 20156A (a 50:50 Butylene glycol:water extract) was shown to inhibit MMP-9 activity by 50% at a concentration of 25μg/ml, and *Tsuga canadensis* extract 20252-PLA inhibited MMP-9 activity by 50% at 156μg/ml.

## EXAMPLE III: *Viability/Growth Assay*

This example provides results reflecting the non-cytotoxic concentration of *Tsuga* extracts that can be used for further *in vitro* efficacy studies.

*Growth evaluation of human skin cells in the presence of* Tsuga *extracts*

Normal human skin fibroblasts and keratinocytes (Cascade Biologics, Portland, OR) were tested to evaluate the anti-proliferative effect of the *Tsuga* extracts. The latter was done to ascertain that the exposure of cells to a concentration of extract would have no undesirable effect for further cellular assays.

Cells (5 X $10^3$ cells/100μl/well for fibroblasts and 8 X $10^3$ cells/100μl/well for keratinocytes) were seeded in a 96-well plate in their respective media and then incubated for 24 hours at 37°C in a humidified 5% $CO_2$ atmosphere. The *Tsuga* extracts were diluted at an initial concentration of 2mg/ml (2X of the final concentration) in appropriate culture media and 4 sequential dilutions were tested for each cell line. Experimental controls were included for each assay: 100μl of media to reflect the maximum growth and viability of cells and 100ng/ml of daunorubicin to obtain an 80% cytotoxic effect. 96-well plates were incubated for 72 hours at 37°C in a humidified 5% $CO_2$ atmosphere. After incubation, Alamar Blue dye was added to each well and fluorescence was read on a Spectrafluor Plus (Tecan, Durham, NC). All assays were done in quadruplicate.

The results for *Tsuga canadensis* extract 207-20156A (from dried needles and small branches) are presented in Figure 1 and are expressed as the % viable cells. The data plotted is the average of the quadruplicate results.

## EXAMPLE IV: *Effect of* Tsuga *Extracts on Angiogenesis*

60 μl Matrigel® was added to a 96-well plate flat bottom plate (Costar 3096) and incubated for 30 minutes at 37°C in a 5% $CO_2$ atmosphere. A mixture of HUVECs and *Tsuga* extract, or a positive control (Fumagillin or GM6001) were added to each well. HUVECs were prepared as suspensions of 2.5 x $10^5$ cells per ml in EGM-2, then 500μl of HUVECs preparation was mixed with 500μl of 2X of the desired dilution of *Tsuga* extract or control drug and 200μl were added to each well. Four dilutions of each extract were tested in duplicate. After 18-24 hours at 37°C in 5% $CO_2$, the cells had migrated and organized into cords.

The number of cell junctions was counted in 3 randomly selected fields and the inhibition of cord formation is calculated as follows:

$$[(A - B)/A] \text{ x } 100,$$

where A is the average number of cell junctions per field in the control well and B is the average number of cell junctions per field in the treated wells.

Following the protocol outlined above, *Tsuga canadensis* extract 20156A inhibited cord formation by 85% at a concentration of 30μg/ml, while Fumagillin at 15μg/ml inhibited cord formation by 100% and GM6001 at 25μM inhibited cord formation by 78%.

## EXAMPLE V: *Inhibition of Contractile Force of Fibroblasts by* Tsuga *Extracts*

The following example demonstrates the ability of *Tsuga* extracts prepared as described in Example I to inhibit contractile force of fibroblasts in an *in vitro* skin model. The skin model comprises human skin fibroblasts imbedded in a collagen I matrix and provides an *in vitro* representation of dermal contraction resulting from tractional forces generated by fibroblasts. Partial or permanent dermal contraction can play a role in the formation of wrinkles. Thus, extracts capable of inhibiting the contractile force of fibroblasts, have the potential to provide a dermo-decontraction anti-ageing effect in the skin.

The ability of the *Tsuga* extracts to inhibit the contractile force of fibroblasts was evaluated on human skin fibroblasts (Cascade Biologics, Portland, OR). The cells were imbedded in a collagen I matrix to create a derm-like environment. Fibroblasts were grown in complete M106 to 80% confluence. Free-floating fibroblast-populated collagen gels were prepared in 24-well plates. 500μl of gel contains 2.5mg/ml of collagen I (rat tail, BD Biosciences, Bedford, MA), M106 5X, NaOH 0.7N; 1X$10^5$ cells and fetal bovine serum (FBS) at 20 % (Wisent, St-Bruno, QC, Canada). The mix was kept on ice until distribution. The derm-like gels were allowed to polymerize for 1 hour at 37°C in a humidified 5% $CO_2$ atmosphere. After incubation, the gels were detached from the wells. Media 106 was used as negative control and GM6001 (Chemicon, Temecula, CA) at a concentration of 50μM was used as positive control. The *Tsuga* extracts were diluted in complete media 106. FBS at a final concentration of 10% was added to each well. The plate was incubated for a maximum of 7 days at 37°C in a humidified 5% $CO_2$ atmosphere. All assays were performed in duplicate. Contraction was measured beginning at day 5. Contracting gels were digitally photographed and the gel areas were calculated using ImagePro software.

Control gels treated with media alone have the smallest area and represent the contracted control. GM6001 was able to provide limited, but not complete, inhibition of contraction. *Tsuga canadensis* extract 20156A inhibits the tractional force of fibroblasts by 103% at a concentration of 100μg/ml and *Tsuga canadensis* extract 20252-PLA inhibits the tractional force of fibroblasts by 128% at a concentration of 40μg/ml, while GM6001 inhibits the tractional force of fibroblasts by 85% at a concentration of 50μM.

**EXAMPLE VI: *Non-Irritating Behaviour of* Tsuga *Extracts***

This example provides results showing the non-irritating quality of the *Tsuga* extracts. The amount of interleukin-8 (IL-8) released in the following assay is used to quantify a possible irritating reaction after exposure of keratinocytes to the *Tsuga* extract.

Release of IL-8 was evaluated on human skin keratinocytes (Cascade Biologics, Portland, OR, catalog number C-005) and measured using the Quantikine hIL8 ELISA kit (R&D Systems, Minneapolis, MN, catalog number D8000C). Keratinocytes were first grown in a 96-well plate using complete medium M154 (M154 + HKGS cat S-001 from Cascade Biologics). This medium was used as a negative (non-irritating) control, while 2.5μM phorbol 12-myristate 13-acetate (PMA) (Sigma-Aldrich Canada, Oakville, Ontario) was used as a positive (irritating) control. All extracts and controls were diluted in complete medium M154. Cells were seeded into 96-well plates at a concentration of 8 X $10^3$cells/well in complete M154 medium and plates were incubated for 48 hours at 37°C in a humidified 5% $CO_2$ atmosphere. The medium was removed and 200μl of *Tsuga* extract or control were added to the wells (all performed in duplicate) and then the plates were incubated for 48 hours at 37°C in a humidified 5% $CO_2$ atmosphere. ELISA was performed using following the manufacturer's recommendations (R&D Systems). The absorbance was read at 450 nm on the Spectrafluor Plus plate reader (Tecan).

Controls treated with M154 medium showed the lowest IL-8 release and this amount was taken as the minimum IL-8 release. PMA induced a strong inflammatory response, which was taken as the highest irritating level (highest IL-8). The evaluation of cytokine release stimulated by a *Tsuga* extract in this experiment enables a maximum concentration of the extract to be set for further *in vivo* studies.

Following the above protocol, *Tsuga canadensis* extract 20252-PLA stimulated 2.2pg/ml of IL-8 release at a concentration of 200μg/ml, while PMA stimulated 282pg/ml of IL-8 release at a concentration of 5μM. The basal level was evaluated at 18pg/ml.

**EXAMPLE VII: *Inhibition of UV-induced proteolytic activity***

This example demonstrates the potential of the *Tsuga* extracts to protect the skin from proteolytic damage after sun exposure.

Fibroblasts were first grown in 24-well plates using complete M106 medium (M06 + LSGS from Cascade Biologics) at a concentration of 6 X $10^4$ cells/500μl/well. The plates were incubated 48 hours at 37°C in a humidified 5% $CO_2$ atmosphere. The medium was removed and the cells were washed 2 times with HBSS. After complete removal of liquid, the cells were irradiated with 25J/cm$^2$ of UVA light. *Tsuga* extracts and controls were added at 500μl/well. The medium was used as a negative (no UV protection) control, while 50μM GM6001 was used as a positive control. All extracts and controls were diluted in complete M106 medium. All assays were done in duplicate except for controls that were done in quadruplicate. Plates were incubated for 24 hours at 37°C in a humidified 5% $CO_2$ atmosphere. Supernatant from each well was assayed or kept at -80°C until use.

The supernatants were assayed for their overall proteolytic activity using the internally quenched peptide, MCA-Pro-Leu-Gly-Dpa-Ala-Arg-NH$_2$ (Bachem, Torrance, CA). Supernatants were distributed in a 96-well black plate, with or without peptide. The reaction took place in a 50mMTris, 150M NaCl, 5mM CaCl$_2$, 0.5mM ZnCl$_2$ pH 7.5 buffer. The incubation was at 37°C. Each of the measurements was done in duplicate. Readings were taken, at 20, 40 and 60 minutes, on the Polarion plate reader (Tecan) with excitation at 330nm and emission at 400nm.

The UVA-irradiated cells showed a higher proteolytic activity than the non-irradiated cells. Inhibition of this enzymatic cleavage could protect against skin damage such as photoaging. Controls treated with medium alone represent the highest proteolytic activity. GM6001 was used as positive control.

Following the above protocol, *Tsuga canadensis* extract 20156A showed good inhibition of the overall proteolytic cleavage. An inhibition of 41% at a concentration of 50μg/ml was evaluated for this extract.

**EXAMPLE VIII: *Anti-inflammatory Effect of* Tsuga *extracts on UVA-Induced Inflammation in Keratinocytes.***

This example provides results showing the anti-inflammatory effect of *Tsuga* extracts when inflammation is induced in keratinocytes. The amount of interleukin-1 alpha (IL-1) released is used to quantify the inflammatory reaction after exposure of the keratinocytes to UVA light irradiation.

IL-1 release was evaluated in human skin keratinocytes (Cascade Biologics, Portland, OR) and was measured using the Quantikine hIL1 ELISA kit (R&D Systems, Minneapolis, MN). Keratinocytes were first grown in a 96-well plate using complete M154 medium with different concentrations of extracts for 48 hours at 37°C in a humidified 5% $CO_2$ atmosphere. All extracts and controls were diluted in complete M154 medium. Cells were rinsed with HBSS and irradiated with UVA at 20J/cm$^2$. HBSS was removed and complete M154 medium containing controls or extracts was added to respective wells. Plates were incubated for another 24 hours.

ELISA was performed following the manufacturer's recommendations (R&D Systems). The absorbance was read at 450 nm on the Spectrafluor Plus plate reader (Tecan).

Controls treated with M154 medium showed the highest inflammatory response and this amount was taken as the highest IL-1 release. Controls in medium without UVA irradiation showed the lowest IL-1 release. The inhibition of IL-1 release is proportional to the anti-inflammatory effect. The inhibition of inflammation by *Tsuga canadensis* extract 207-20156A according to this protocol is shown in Figure 2.

As shown in Figure 2, even at 0.012 mg/mL *Tsuga canadensis* extract 207-20156A inhibited IL-1 release by 53%, whereas salicylic acid, which is a well-known anti-inflammatory agent, inhibited IL-1 release by 11% at 0.025% and by 27% at 0.05%.

**EXAMPLE IX: *Effect of* Tsuga *Extracts on the Expression of Psoriasis Markers in Keratinocytes***

This Example provides results showing the effect of *Tsuga* extracts on the expression of different markers expressed by psoriasis-like keratinocytes. Cell markers such as cytokeratine-16 (CK16), CK10, transglutaminase II, involucrin and SKALP are over-expressed in keratinocytes that are involved in psoriasis. Induction of cornification and differentiation in keratinocytes results in over-expression of these markers, mimicking the psoriatic state. The *Tsuga canadensis* extract used in this Example (*Tsuga canadensis* extract 9338 4AC022C4) was an alcoholic extract prepared by extracting 10g of dried plant material from *Tsuga canadeusis* lot 9338 with 100ml of Ethanol:MeOH 85:15, following the general protocol outlined in Example I.

Human skin keratinocytes (Cascade Biologics, Portland, OR) were first grown in 96-well plates using complete M154 medium with various concentrations of *Tsuga canadensis* extract 9338 for 72 hours at 37°C in a humidified 5% $CO_2$ atmosphere. Differentiation was initiated with 10% fetal calf serum added to the well at the start of the incubation period. The cells were rinsed with HBSS and fixed with a 70:30 (ethanol:acetone) solution. After rinsing with PBS, 4% dried milk in PBS was added to each well and the plate incubated for 1 hour at 37°C. Each of the antibodies anti-CK10, anti-CK16, anti-involucrin, anti-SKALP and anti-transglutaminase II was diluted, as recommended by the manufacturer, in PBS containing 1% dried milk, added to the wells and the plate incubated for 1 hour at 37°C. Four washes were performed with PBS-0.05% Tween prior to the addition of the secondary antibody: anti-IgG labelled with Alexa 594 or Alexa 485 (Molecular Probes) diluted in PBS with 1% dried milk to the recommended concentration. The plate was then incubated for an additional 1 hour at 37°C. Four washes were then performed prior to evaluation under a fluorescent microscope. The results are shown in Table I and show that the effect of *Tsuga canadensis* extract 9338 4AC022C4 on the expression of the markers was comparable to that of the positive control Dithranol, a known anti-psoriasis agent.

**Table I: Evaluation of the Effect of *Tsuga canadensis* extract 9338 4AC022C4 on Expression of Psoriasis Markers**

| No induction | - | +++ | +++ | +++ | +++ | +++ |
|---|---|---|---|---|---|---|
| Induced cells | - | - | - | - | - | - |
| Dithranol | 4.5μg/ml | +/- | + | ++ | +/- | + |
| *Tsuga* 9338 4AC022C4 | 200μg/ml | +/- | ++ | ++ | + | + |

**EXAMPLE X: *Anti-inflammatory Effect of* Tsuga *Extracts in Humans: A Case Report***

*Materials and method*

Four creams were prepared using a 50:50 butylene glycol:water *Tsuga canadensis* extract prepared as described in Example I. The creams were as follows (all % are w/w):

Cream #1: 0.5% retinol
Cream #2: 0.5% retinol and 5% *Tsuga canadensis* extract
Cream #3: 1% retinol
Cream #4: 1% retinol and 5% *Tsuga canadensis* extract

The patient was a healthy female volunteer age 39. She applied all four creams (#1 to #4, above) twice a day in a blinded manner to her forearms. She noted any sign of irritation. The study was stopped when the redness due to irritation was too strong and discomfort appeared.

*Results*

The results after 21 days of treatment are shown in Figure 3. An irritating effect of retinol at 1% (Cream #3) was observed starting at day 10 of the experiment. The redness was intense at day 18 to 21, when the patient was told to stop the study. The *Tsuga* extract at 5% (Cream #4) clearly helped to diminish the level of inflammation caused by retinol from an assessment of "3" to an assessment of "1" based on qualitative evaluation of the redness of the skin. The intensity of inflammation was less when the 0.5% retinol cream (Cream #1) was used. However, addition of the *Tsuga* extract at 5% (Cream #2) also helped to diminish the level of inflammation caused by this lower amount of retinol.

**EXAMPLE XI:** *Determination of Irritation or Sensitization by* Tsuga *Extracts on Human Skin I*

This example and Example XIV below demonstrate that the *Tsuga* extracts are non-irritating to human skin. The test employed was the HRIPT (Human Repeat Insult Patch Test), which determines if a topical agent has the potential to induce irritation or sensitization of any kind.

52 volunteers were selected for the study. The volunteers were men and women of ages 23 to 57. The agent, *Tsuga canadensis* extract 20252-PLA 5% (v/v) in petroleum jelly, was applied to the skin of the volunteers repeatedly using 10 patches over a period of 3 weeks. The patches used in the study were TruMed® semi-occlusive, cotton "Nova® net embossed" with "Avery® 4750U tape" adhesive backing. After a rest period (incubation phase) varying from 10 to 14 days, a challenge phase was conducted. The patch was applied for 48 hours and removed. The test sites were cleaned and examined for any signs of intolerance or irritation by a dermatologist.

*Results:*

Under the conditions of the study, *Tsuga canadensis* extract 20252-PLA produced no signs of cutaneous irritation or skin sensitization in either the induction phase or the challenge phase of the test. The extract is therefore considered non-irritant and potentially hypo-allergenic. In addition, given the control provided by a dermatologist, the test product may bear the claim "Tested under control of a dermatologist."

**EXAMPLE XII:** *Anti-microbial Activity of* Tsuga *Extracts I*

To determine the antimicrobial effect of *Tsuga* extracts, *Escherichia coli* (ATCC 8739), *Staphylococcus aureus* (ATCC 6538) and *Pseudomonas aeruginosa* (ATCC 9027) were used to challenge different concentrations of *Tsuga canadensis* extract 20156A (diluted in water). *E. coli* was inoculated at 1.5 X $10^6$ bacteria/ml in 10ml of diluted extract; *S. aureus* was inoculated at 2 X $10^6$ bacteria/ml in 10ml of diluted extract and *P. aeruginosa* was inoculated at 1 X $10^6$ bacteria/ml in 10ml of diluted extract. All samples of extract or vehicle (50:50 butylene glycol:water) were incubated at 37°C and evaluated at 0, 7, 14 and 28 days. The effectiveness was analyzed by determining the colony forming unit (CFU) per g on tryptic soy agar.

The results are summarized in Tables II A, B and C and demonstrate that when the *Tsuga* extract was challenged with 3 different species of bacteria, a significant inhibition of growth of the bacteria resulted even after the first incubation period.

**Table II. Evaluation of the antimicrobial effect of *Tsuga canadensis* extract 20156A against *E. coli, S. aureus* and *P. aeraginosa* (results in CFU)**

| A. E. coli | | | | |
|---|---|---|---|---|
| | | | | |
| *Tsuga* extract 20156A 100% | 5.1E+03 | 180 | 0 | 0 |
| *Tsuga* extract 20156A 50% | 2.2E+05 | 100 | 0 | 0 |
| *Tsuga* extract 20156A 25% | 3.6E+05 | 50 | 0 | 0 |

(continued)

| A. *E*. *coli* | | | | |
|---|---|---|---|---|
| *Tsuga* extract 20156A 10% | 3.7E+05 | 5.3E+03 | 0 | 0 |
| Vehicle 100% | 1.7E+05 | 0 | 0 | 0 |
| Vehicle 50% | 3.5E+05 | 50 | 0 | 0 |
| Vehicle 25% | 3.6E+05 | 6.1E+05 | 9.0E+04 | 0 |
| Vehicle 10% | 3.2E+05 | 9.0E+06 | 7.7E+08 | 1.2E+09 |
| Bacteria + Nutrient Broth | 3.3E+05 | 7.7E+12 | 5.9E+15 | 2.0E+14 |
| **B. *S*. *aureus*** | | | | |
| | | | | |
| *Tsuga* extract 20156A 100% | 0 | 0 | 0 | 0 |
| *Tsuga* extract 20156A 50% | 0 | 0 | 0 | 0 |
| *Tsuga* extract 20156A 25% | 0 | 0 | 0 | 0 |
| *Tsuga* extract 20156A 10% | 15 | 0 | 0 | 0 |
| Vehicle 100% | 4.8E+02 | 0 | 0 | 0 |
| Vehicle 50% | 5.5E+04 | 0 | 0 | 0 |
| Vehicle 25% | 4.5E+04 | 4.2E+06 | 1.7E+07 | 2.4E+06 |
| Vehicle 10% | 8.1E+04 | 3.7E+06 | 9.6E+07 | 9.0E+05 |
| Bacteria + Nutrient Broth | 4.8E+04 | 2.5E+13 | 5.0E+11 | 2.6E+14 |
| **C. *P*. *aeruginosa*** | | | | |
| | | | | |
| *Tsuga* extract 20156A 100% | 4.5E+03 | 0 | 0 | 0 |
| *Tsuga* extract 20156A 50% | 1.0E+03 | 0 | 0 | 0 |
| *Tsuga* extract 20156A 25% | 5.5E+03 | 0 | 0 | 0 |
| *Tsuga* extract 20156A 10% | 1.1E+04 | 50 | 0 | 0 |
| Vehicle 100% | 2.0E+03 | 0 | 0 | 0 |
| Vehicle 50% | 1.4E+04 | 0 | 0 | 0 |
| Vehicle 25% | 7.5E+03 | 3.1E+02 | 0 | 0 |
| Vehicle 10% | 8.0E+03 | 3.9E+04 | 1.4E+04 | 0 |
| Bacteria + Nutrient Broth | 1.1E+04 | 9.4E+12 | 5.1E+12 | 5.3E+14 |
| [1] All % are v/v. | | | | |

**EXAMPLE XIII:** *Exploratory Study of the Anti-Psoriatic Effect of a Dermological Formulation of a* Tsuga *Extract*

Four volunteers were selected for the study. The volunteers were men and women of ages over 18 with mild to moderate psoriatic lesions (plaques). The agent, *Tsuga canadensis* extract 5% (v/v) formulated in a cream (Lotion Glaxal base from Wellskin), was applied onto the plaque twice a day for a period of 8 weeks. A placebo was applied to a similar plaque. The patients received two bottles in a blinded manner and were required to fill out a follow-up sheet. Patients had a follow-up evaluation by a dermatologists at weeks (W) 0, 4 and 8, in which the lesions were scored for erythema (E), squame or dryness (S), thickness or induration (I) and surface (A). The results are presented in Tables IIIA and B below.

**Table III: Evaluation of Psoriatic Lesions Treated with 5% *Tsuga canadensis* Extract after 8 Weeks**

| A. *Tsuga canadensis* Extract | | | | | |
|---|---|---|---|---|---|
| **Lesion or plaque** | **W0** | **W4** | **W8** | **△W4-W0** | **△W8-W0** |
| Squames (S) | 12 | 8 | 7 | -33% | |
| Erythema (E) | 13 | 11 | 10 | -15% | |
| Thickness (I) | 12 | 10 | 9 | -17% | -25% |
| Surface (A) | 9 | 9 | 8 | 0% | -11% |
| Total | 46 | 38 | 34 | -17% | -26% |
| **B. Placebo** | | | | | |
| **Lesion or plaque** | **W0** | **W4** | **W8** | **△W4-W0** | **△W8-W0** |
| Squames (S) | 13 | 9 | 8 | -31% | -38% |
| Erythema (E) | 13 | 12 | 11 | -8% | -15% |
| Thickness (I) | 12 | 9 | 9 | -25% | -25% |
| Surface (A) | 13 | 13 | 12 | 0% | -8% |
| Total | 51 | 43 | 40 | -16% | -22% |

Under the conditions of the study, the *Tsuga canadensis* extract at 5% (v/v) in a dermalogical formulation produced a beneficial effect on psoriatic lesions. The effect was primarily on two parameters: erythema (E, redness) and squame (S). The results are significant enough to warrant further clinical studies.

**EXAMPLE XIV:** *Determination of Irritation or Sensitization by* Tsuga *Extracts on Human Skin II*

54 volunteers were selected for the study. The volunteers were men and women of ages 20 to 53. The agent, *Tsuga canadensis* extract 20376 1NG23ZB 10% (v/v) in a base cream, was applied to the skin of the volunteers repeatedly using 10 patches over a period of 3 weeks. The patches used in the study were TruMed® semi-occlusive, cotton "BBA149-129 Absorbent" with "3M1530 tape" adhesive backing. After a rest period (incubation phase) varying from 10 to 14 days, a challenge phase was conducted. The patch was applied for 48 hours and removed. The test sites were cleaned and examined for any signs of intolerance or irritation by a dermatologist.

*Results:*

Under the conditions of the study, *Tsuga canadensis* extract 20376 1NG23ZB 10% (v/v) produced no signs of cutaneous irritation or skin sensitization in either the induction phase or the challenge phase of the test. The extract is therefore considered non-irritant and potentially hypo-allergenic. In addition, given the control provided by a dermatologist, the test product may bear the claim "Tested under control of a dermatologist."

**EXAMPLE XV: Anti-microbial Activity of** Tsuga *Extracts II*

To determine the antimicrobial effect of *Tsuga* extracts, *Escherichia coli* (ATCC 8739), *Staphylococcus aureus* (ATCC 6538) and *Pseudomonas aeruginosa* (ATCC 9027) were used to challenge different concentrations of *Tsuga canadensis* extract 20010 1NG13B (diluted in water). *E. coli* was inoculated at $1.5 \times 10^6$ bacteria/ml in 10ml of diluted extract; *S. aureus* was inoculated at 2 X 10$^6$ bacteria/ml in 10ml of diluted extract and *P. aeruginosa* was inoculated at 1 X 10$^6$ bacteria/ml in 10ml of diluted extract. All samples of extract or vehicle (50:50 butylene glycol:water) were incubated at 37°C and evaluated at 0, 7, 14 and 28 days. The effectiveness was analyzed by determining the colony forming unit (CFU) per g on tryptic soy agar.

The results are summarized in Tables IV A, B and C and demonstrate that when the *Tsuga* extract was challenged with 3 different species of bacteria, it showed a significant inhibition of growth even after the first incubation period.

**Table IV. Evaluation of the antimicrobial effect of *Tsuga canadensis* extract 20010 1NG13B against *E. coli*, *S. aureus* and *P. aeruginosa* (results in CFU)**

| A. *E. coli* | | | | |
|---|---|---|---|---|
| *Tsuga* extract 20010 1NG13B 10% | 2.44E+05 | 0 | 0 | 0 |
| *Tsuga* extract 20010 1NG13B 5% | 3.29E+05 | 0 | 0 | 0 |
| *Tsuga* extract 20010 1NG13B 1% | 2.81E+05 | 0 | 0 | 0 |
| Vehicle 10% | 2.78E+05 | 0 | 0 | 0 |
| Vehicle 5% | 2.95E+05 | 5.56E+07 | 3.40E+09 | 5.60E+05 |
| Vehicle 1% | 3.72E+05 | 6.21E+07 | 4.20E+09 | 7.60E+07 |
| Bacteria + Nutrient Broth | 3.27E+05 | 3.96E+11 | 4.50E+14 | 1.29E+17 |
| B. *S. aureus* | | | | |
| | | | | |
| *Tsuga* extract 20010 1NG13B 10% | 1.00E+04 | 0 | 0 | 0 |
| *Tsuga* extract 20010 1NG13B 5% | 9.10E+04 | 0 | 0 | 0 |
| *Tsuga* extract 20010 1NG13B 1% | 1.38E+05 | 7.75E+07 | 4.60E+09 | 2.40E+07 |
| Vehicle 10% | 1.35E+05 | 5.20E+06 | 7.00E+08 | 5.00E+07 |
| Vehicle 5% | 1.10E+05 | 2.85E+07 | 6.00E+08 | 3.00E+07 |
| Vehicle 1% | 1.30E+05 | 2.77E+07 | 6.00E+08 | 2.00E+10 |
| Bacteria + Nutrient Broth | 1.47E+05 | 8.01E+11 | 9.08E+15 | 2.26E+17 |
| C. *P. aeruginosa* | | | | |
| | | | | |
| *Tsuga* extract 20010 1NG13B 10% | 1.88E+05 | 2.00E+01 | 0 | 0 |
| *Tsuga* extract 20010 1NG13B 5% | 1.26E+05 | 6.83E+07 | 2.25E+10 | 2.94E+09 |
| *Tsuga* extract 20010 1NG13B 1% | 1.14E+05 | 6.83E+07 | 7.35E+10 | 2.34E+09 |
| Vehicle 10% | 1.29E+05 | 4.02E+07 | 7.40E+09 | 1.92E+09 |
| Vehicle 5% | 1.49E+05 | 7.87E+07 | 2.32E+10 | 1.22E+09 |
| Vehicle 1% | 8.70E+04 | 7.10E+07 | 6.74E+10 | 1.25E+09 |

(continued)

| C. *P. aeruginosa* | | | | |
|---|---|---|---|---|
| Bacteria + Nutrient Broth | 1.54E+05 | 5.52E+11 | 4.35E+15 | 2.00E+17 |
| [1] All % are v/v. | | | | |

### EXAMPLE XVI: Exemplary Dermatological Formulations Comprising a Tsuga Extract

The following formulation comprising a *Tsuga canadensis* extract is a non-limiting example of a suitable formulation for human efficacy studies (such as those described in Examples XVII and XVIII). The formulation can be modified for commercial use as is known in the art. All % are w/w.

| Cream: | Beeswax | 1-2% |
|---|---|---|
| | Glycerin | 2-4% |
| | Xanthan Gum | 0.5-1% |
| | PPG-15 stearyl ether | 6-10% |
| | Stearic acid | 1-2% |
| | Kaleol 8670 | 1-4% |
| | EDTA | 0.25-0.5% |
| | Shea butter | 1-3% |
| | Extract 20252 | 0.1-5% |
| | Preservatives | 0.2-1% |
| | Water | q.s. 100% |

Other examples of formulations comprising *Tsuga canadensis* extracts include those in which the extract is added in an amount between 0.1 and 10% (w/w) into a pre-made skin formulation. For example, a cream base, a lotion, an aftershave lotion, a soothing mask, or petrolatum jelly.

### EXAMPLE XVII: *Evaluation of Wrinkle Attenuation by the* Tsuga *Extracts*

The following Example provides an exemplary protocol that may be followed to evaluate the ability of a *Tsuga* extract to improve the appearance of wrinkles in human volunteers.

Healthy volunteers are divided in 2 groups. Each group of volunteers receives a composition containing 5% (v/v) *Tsuga* extract. The composition is applied around the eyes twice a day for a period of 12 weeks. Each volunteer is asked to comment on the composition and the improvement they feel in their wrinkles with a scale from -3 (greatly worsened) to 0 (no change) to +3 (greatly improved).

The improvement in the appearance of the wrinkles can also be determined by profilometry. In this case, replicas (negatives of the skin surface) of the eye contour zones of the volunteers are obtained prior to and after treatment by applying silicone polymer onto the "crow's feet" of the eye contour zone, while the volunteer maintains an upright but sitting position. Replicas of the crow's feet are then analyzed by a computerized digital image processing system coupled to Quantirides® software to obtain the skin's topography. This standard technique is based on measuring the shadows cast when an incident light is inclined at an angle of 35° on the replica.

The analyzed parameters are: the total area of wrinkled skin, the number and the mean depth of the depressions due to the cutaneous relief, and depth of deep and medium-deep wrinkles. The wrinkles are differentiated by classes of depth (Class 1 for 0-55mm; Class 2 for 55-110 mm; and Class 3 for 110- 800 mm).

### EXAMPLE XVIII: *Evaluation of the Anti-Inflammatory Activity of the* Tsuga *Extracts in Humans*

The following example provides an exemplary protocol for assessing the ability of the *Tsuga* extracts to attenuate UV-induced erythema.

The MED test measures the minimum UV dose required to produce a distinct reaction in the form of redness that appears 24 hours after exposure. The MED of all volunteers must be measured prior to any treatment that attempts to attenuate the skin response to UV-induced erythema. Accordingly, at Day D0, the skin color for each volunteer is measured in the test zone (forearm) using a Mexameter® to approximate the MED of the individual volunteer. Actinic erythema is induced using a UV simulator to the selected zone by a series of 5 consecutive increasing irradiations on a non-treated site on the right forearm and on a site treated with the extract on the left forearm. The skin response on each forearm is then assessed 24 hrs later (Day D1) to calculate the individual MED (the lowest dose to which a distinct reaction is observed). The change observed with the extract can be scored, for example, on a numerical scale in which 5 indicates "very improved" and 0 indicates "no change." The ability of the extract to decrease the observed redness is indicative of its anti-inflammatory effect.

## Claims

1. A butylene glycol:water 50:50 (v/v) extract from needles, twigs or small branches of a *Tsuga canadensis* plant, for use to treat microbial infection of the skin in a subject in need thereof, wherein the microbial infection of the skin is associated with acne.

2. The extract for use according to claim 1 wherein said extract is incorporated into a dermatological formulation for topical administration.

3. The extract for use according to claim 2, wherein the dermatological formulation is a cosmetic or an anti-acne product.

4. The extract for use according to claim 2 or 3, wherein the dermatological formulation is formulated for sensitive skin.

5. The extract for use according to claim 1, wherein the use is in combination with one or more other anti-inflammatory agents, a retinol or a retinol derivative.

6. A dermatological formulation comprising an extract as defined in any one of claims 1 to 5, in combination with one or more other therapeutic or cosmetic agents, for use according to claim 1.

7. The dermatological formulation for use according to claim 6, wherein the dermatological formulation comprises an anti-inflammatory agent or a retinol derivative.

8. The dermatological formulation for use according to claim 6 or 7, wherein the extract is present in an amount between 0.1% and 20% by weight.

9. The dermatological formulation for use according to any one of claims 6, 7 or 8, wherein the dermatological formulation is a cosmetic.

10. The dermatological formulation for use according to claim 6, wherein the one or more other therapeutic or cosmetic agents is an anti-inflammatory agent, retinol, a retinol derivative, an alpha-hydroxy acid and/or a skin whitening agent.

11. The dermatological formulation for use according to claim 7, wherein the anti-inflammatory agent is salicylic acid, and said retinol derivative is tretinoin or isotretinoin.

## Patentansprüche

1. Butylenglykol:Wasser- 50:50 (v/v) -Extrakt aus Nadeln, Zweigen oder kleinen Ästen einer *Tsuga canadensis-Pflanze,* zur Verwendung bei der Behandlung einer mikrobiellen Infektion der Haut bei einem Individuum, das dessen bedarf, wobei die mikrobielle Infektion der Haut mit Akne in Verbindung steht.

2. Extrakt zur Verwendung gemäß Anspruch 1, wobei dieser Extrakt in eine dermatologische Formulierung zur topischen Verabreichung aufgenommen ist.

3. Extrakt zur Verwendung gemäß Anspruch 2, wobei die dermatologische Formulierung ein kosmetisches oder ein Anti-Akne-Produkt ist.

4. Extrakt zur Verwendung gemäß Anspruch 2 oder 3, wobei die dermatologische Formulierung für empfindliche Haut formuliert ist.

**5.** Extrakt zur Verwendung gemäß Anspruch 1, wobei die Verwendung in Kombination mit ein oder mehreren anderen entzündungshemmenden Mitteln, einem Retinol oder einem Retinol-Derivat erfolgt.

**6.** Dermatologische Formulierung, umfassend einen Extrakt, wie in einem der Ansprüche 1 bis 5 definiert, in Kombination mit ein oder mehreren anderen therapeutischen oder kosmetischen Mitteln, zur Verwendung gemäß Anspruch 1.

**7.** Dermatologische Formulierung zur Verwendung gemäß Anspruch 6, wobei die dermatologische Formulierung ein entzündungshemmendes Mittel oder ein Retinol-Derivat umfasst.

**8.** Dermatologische Formulierung zur Verwendung gemäß Anspruch 6 oder 7, worin der Extrakt in einer Menge von zwischen 0,1 und 20 Gewichts-% vorhanden ist.

**9.** Dermatologische Formulierung zur Verwendung gemäß einem der Ansprüche 6, 7 oder 8, wobei die dermatologische Formulierung ein Kosmetikum ist.

**10.** Dermatologische Formulierung zur Verwendung gemäß Anspruch 6, worin die ein oder mehreren anderen therapeutischen oder kosmetischen Mittel ein entzündungshemmendes Mittel, Retinol, ein Retinol-Derivat, eine alpha-Hydroxysäure und/oder ein Hautbleichmittel sind.

**11.** Dermatologische Formulierung zur Verwendung gemäß Anspruch 7, worin das entzündungshemmende Mittel Salicylsäure ist und das Retinol-Derivat Tretinoin oder Isotretinoin ist.

**Revendications**

**1.** Extrait par butylène glycol:eau 50:50 (v/v) à partir d'aiguilles, de brindilles ou de petites branches d'une plante *Tsuga canadensis,* pour une utilisation pour traiter une infection microbienne de la peau chez un sujet en ayant besoin, l'infection microbienne de la peau étant associée à l'acné.

**2.** Extrait pour une utilisation selon la revendication 1, dans lequel ledit extrait est incorporé dans une formulation dermatologique pour une administration topique.

**3.** Extrait pour une utilisation selon la revendication 2, dans lequel la formulation dermatologique est un produit cosmétique ou un produit anti-acné.

**4.** Extrait pour une utilisation selon l'une des revendications 2 ou 3, dans lequel la formulation dermatologique est formulée pour une peau sensible.

**5.** Extrait pour une utilisation selon la revendication 1, dans lequel l'utilisation est en combinaison avec un ou plusieurs autres agents anti-inflammatoires, un rétinol ou un dérivé de rétinol.

**6.** Formulation dermatologique comprenant un extrait tel que défini dans l'une quelconque des revendications 1 à 5, en combinaison avec un ou plusieurs autres agents thérapeutiques ou cosmétiques, pour une utilisation selon la revendication 1.

**7.** Formulation dermatologique pour une utilisation selon la revendication 6, dans laquelle la formulation dermatologique comprend un agent anti-inflammatoire ou un dérivé de rétinol.

**8.** Formulation dermatologique pour une utilisation selon l'une des revendications 6 ou 7, dans laquelle l'extrait est présent dans une quantité entre 0,1 % et 20 % en poids.

**9.** Formulation dermatologique pour une utilisation selon l'une quelconque des revendications 6, 7 ou 8, dans laquelle la formulation dermatologique est un produit cosmétique.

**10.** Formulation dermatologique pour une utilisation selon la revendication 6, dans laquelle le ou les autres agents thérapeutiques ou cosmétiques sont un agent anti-inflammatoire, le rétinol, un dérivé de rétinol, un alpha-hydroxy acide et/ou un agent de blanchiment de la peau.

11. Formulation dermatologique pour une utilisation selon la revendication 7, dans laquelle l'agent anti-inflammatoire est l'acide salicylique, et ledit dérivé de rétinol est la trétinoïne ou l'isotrétinoïne.

**FIGURE 1**

**FIGURE 2**

**FIGURE 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5141741 A
- US 6764693 B
- US 5837224 A
- US 6130254 A
- US 6365630 B
- US 20010053347 A
- US 6307101 B
- US 4857325 A
- US 6342208 B
- US 4923697 A
- US 6800292 B
- US 6682763 B
- US 5824320 A
- US 6406720 B
- US 5073545 A
- US 6676975 B
- US 4855131 A
- US 6193975 B
- CA 0402007 W
- WO 2006053415 A
- US 2276241 A
- GB 544615 A
- CA 406408
- JP 2002087973 B
- JP 4030855 B
- US 4898727 A
- JP 0870507 A
- WO 0206992 A

### Non-patent literature cited in the description

- **WELSS T.** *Toxicology in vitro,* 2004, vol. 18, 231
- **KIM BH.** *Toxicology Letters,* 2003, vol. 146, 65
- **BOEHM B.** *Exp Opinion Invest Drugs,* 1995, vol. 4, 593
- **VARANI J.** *J Invest Dermatol Symp,* 1998, vol. 3, 57
- **KLIGMAN AM.** *J Dermatol Threat,* 1999, vol. 10, 119
- **MARTINEZ, J.L.** supercritical Fluid Extraction of Nutraceuticals and Bioactive Compounds. CRC Press, 2007
- **HERRERO. M. et al.** *Food Chem,* 2005, vol. 98, 136-148
- **AGNER, T.** *Acta Derm Venereol Suppl (Stockh),* 1992, vol. 173, 1-26
- **DE FINE OLIVARIOUS, F. et al.** *Br J Dermatl.,* 1993, vol. 129, 554-557
- Susceptibility testing of antimicrobials in liquid media. **AMSTERDAM, D.** Antibiotics in Laboratory Medicine. Williams and Wilkins, 1996, 52-111
- **JAFFE et al.** *J. Clin. Invert.,* 1973, vol. 52 (11), 2745-56
- **MURPHY ; CRABBE.** Methods in Enzymology. Proteolytic Enzymes: Aspartic Acid and Metallopeptidases. Academic Press, 1995, vol. 248, 470
- **PACMEN et al.** *Biochem. Pharm.,* 1996, vol. 52, 105-111
- **NAGASE ; FIELDS.** *Biopolymers,* 1996, vol. 40, 399-416
- **ST-PIERRE et al.** *Cytometry,* 1996, vol. 25, 374-380
- **NISHIMORI et al.** *J. Invert. Dermatol.,* 2001, vol. 117, 1458-1463
- **I.M. CLARK.** Matrix metalloproteinases protocols. Humana Press, 2001